(19) Europäisches Patentamt
European Patent Office
Office européen des brevets

(11) Publication number: **0 353 777 A2**

# (12) EUROPEAN PATENT APPLICATION

(21) Application number: 89114482.6

(22) Date of filing: 04.08.89

(51) Int. Cl.[4] **C07D 417/12 , C07D 405/12 , A61K 31/425 , A61K 31/44**

Claim for the following Contracting State: ES.

(30) Priority: 04.08.88 ES 8802446

(43) Date of publication of application: 07.02.90 Bulletin 90/06

(84) Designated Contracting States: AT BE CH DE FR GB GR IT LI LU NL SE

(71) Applicant: **J. URIACH & CIA. S.A.**
Degà Bahi, 59-67
E-08026 Barcelona(ES)

(72) Inventor: **Bartroli, Javier**
Vives i Tutó 55
E-08034 Barcelona(ES)
Inventor: **Anguita, Manuel**
Rambla Volart 85
E-08026 Barcelona(ES)
Inventor: **Carceller, Elena**
Aragón 117
E-08015 Barcelona(ES)

(74) Representative: **Zumstein, Fritz, Dr. et al**
Zumstein & Klingseisen Patentanwälte
Bräuhausstrasse 4
D-8000 München 2(DE)

## (54) New 2,5-disubstituted tetrahydrofuran derivatives.

(57) The present invention relates to new derivatives of 2,5-disubstituted tetrahydrofurans with a potent antagonist activity of the platelet activating factor (PAF), together with a process for their preparation. The invention also relates to the pharmaceutical preparations which contain these compounds an their use in the treatment of diseases in which PAF is involved, such as allergic and bronchial asthma, platelet aggregation disorders, septic shock, hypertension, etc.



Xerox Copy Centre

# New 2,5-disubstituted tetrahydrofuran derivatives.

## FIELD OF THE INVENTION

The present invention relates to new derivatives of 2,5-disubstituted tetrahydrofurans with a potent antagonist activity of the platelet activating factor (PAF), together with a process for their preparation. The invention also relates to the pharmaceutical preparations which contain these compounds an their use in the treatment of diseases in which PAF is involved, such as allergic and bronchial asthma, platelet aggregation disorders, septic shock, hypertension, etc.

## BRIEF DESCRIPTION OF THE PRIOR ART

The platelet activating factor (PAF) or (1-*O*-alkyl-2-acetyl-*sn*-glyceryl-3-phosphorylcholine), also called acetyl glyceryl ether phosphorylcholine (AGEPC) or PAF-acether is a natural phospholipid synthesized by different cells (basophiles, macrophages, neutrophiles, platelets) and tissues (heart, lung and kidney) of the organism (Roubin et al. in "Lymphokines' Ed. E. Pick. Acad. Press. New York, p. 249, **1983**; Vargaftig et al, *Ann. N.Y. Acad. Sci.*, **1981**, 370, 119; Pinckard et al., *Int. Arch. Allergy Appl. Immun.*, **1981**, 66, 127).

PAF was described for the first time as a potent platelet aggregating agent (Benveniste et al., *J. Exp. Med.*, **1972**, 136) and later it was demonstrated to have other biological activities *in vivo*, such as peripheral vasodilatation, increase of the vascular permeability, induction of bronchoconstriction and hyperreactivity of the respiratory tract (Mazzoni et al. Proc. Physiol. Soc. Univ. Coll. Meet., March 1985). PAF also produces immediate hypotension followed by pulmonary and renal hypertension in rats (Blank et al., *Biochem. Biophis. Res. Commun.*, **1979**, 90, 1194), guinea pigs (Feuerstein, et al., *Circul. Shock*, **1984**, 13, 255), rabbits (Muirhead et al., *Hypertension*, **1981**, 3, 107) and dogs (Otsuka et al., *J. Exp. Med.*, **1972**, 136, 1356), and it has been rated as the most potent ulcerogenic agent described until now.

Consequently, PAF is a mediator that is implicated in a large set of pathological processes such as asthma, septic shock, transplant rejection, thrombosis, ulceration, inflammation and renal diseases.

Even though its mechanism of action is still not known with precision, some studies show that the biological activities of PAF involve the existence of a specific receptor. Recently, it has been possible the isolation of one of these receptors from human platelets and it has been identified as a protein with a molecular weight of 160.000 daltons (Nishihira et al., *Tohoku J. Exp. Med.*, **1985**, 147, 145). On the other hand, the capacity to inhibit the binding of $^3$H-PAF to their receptors is well correlated with the amount of PAF needed to provoke the *in vitro* and *in vivo* observed effects. These facts indicate that the compounds that act as specific antagonists of PAF could result of interest for the treatment of all those pathological processes related directly or indirectly with PAF.

Until now, several PAF analoges have been investigated in order to find compounds with the antagonist activity above mentioned, for example, the compounds described in patents EP 147768, EP 146258, EP 138559, EP 157609, JP 57/165394, JP 58/133116, JP 58/35116, EP 209239, WO 86/01507, EP 210804, EP 0178261, among others. Patent EP 144804 describes 2,5-diaryltetrahydrofurans with antagonist action of PAF. These compounds, however, are structurally different to those of the present invention due to the aromatic character of their substituents. On the other hand, patent EP 178261, describes 2,5-disubstituted tetrahydrofurans as PAF antagonists; in these compounds one of the substituents is a group of formula-$(CH_2)_nOP(=O)(O^-)O-(CH_2)_m-\overset{+}{N}R_1R_2R_3$ wherein $n=1$-$2$, $m=2$-$8$ and $R_1R_2R_3$ are alkyl substituents or $\overset{+}{N}R_1R_2R_3$ is an heterocycle. The compounds of the present invention not only are structurally different due to the fact that do not carry a phosphate function in their skeleton but also, they show a remarkably higher PAF-antagonist activity.

## DESCRIPTION OF THE INVENTION

The present invention relates to 2,5-disubstituted derivatives of tetrahydrofuran having the general formula **I**:

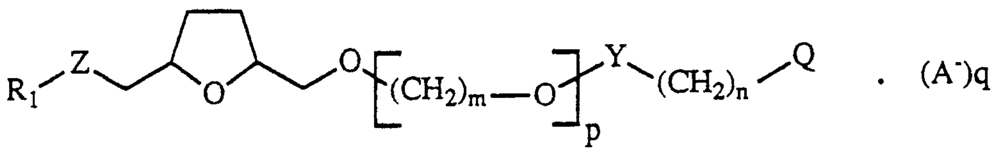

I

where:

$-R_1$ represents a $C_{10}$-$C_{24}$ alkyl, alkenyl or alkynyl group;

-Z- represents either a covalent single bond or a group of formula -O-, -C(=O)O-, -OC(=O)O-, or $-NR_2C$-(=O)O-, where $R_2$ is hydrogen, $C_1$-$C_4$ alkyl, $C_1$-$C_4$ acyl, haloacetyl, or $C_1$-$C_4$ alkoxycarbonyl;

-Y- is either a covalent single bond or a -C(=O) or $-C(=O)NR_2$-group;

-n is an integer from 0 to 10;

-m is 0 or represents an integer from 2 to 8;

-p is 0 when m is 0 and p is 1 when m is not 0.

-Q means:

either a non-charged heterocyclic radical that contains a non-quaternary nitrogen atom which is linked to the alkylene chain by the ring carbon (in which case, -Q is represented by $-Q'$ and q=0),

or the same radical $Q'$, already quaternized (in which case -Q is represented $-Q^{'(+)}$ by and q=1),

or an heterocyclic radical linked to the chain by a quaternary nitrogen (in which case -Q is represented by $Q^{'''(+)}$ and q=1).

-Q can contain additional N atoms, one or more sulphur or oxygen atoms, and can be substituted by one or several $C_1$-$C_5$ alkyl, $C_1$-$C_5$ alkoxy, carbamoyl, or $C_1$-$C_6$ hydroxyalkyl groups, or halogen.

$A^-$ represents a pharmaceutically acceptable anion, such as halide (chloride, bromide or iodide), $C_1$-$C_{10}$ alkylsulfonate, arylsulfonate or carboxylate.

Compounds of formula I have at least two asymmetric carbons, which can give rise to stereoisomers. The present invention includes these stereoisomers as well as their mixtures.

Although the present invention includes all of the above mentioned compounds, the following are specially preferred:

In the compounds of general formula I, preferred examples of the $R_1$ group are *n*-tridecyl, *n*-tetradecyl, *n*-pentadecyl, *n*-hexadecyl, *n*-heptadecyl, *n*-octadecyl and *n*-nonadecyl, and still more preferred are *n*-pentadecyl, *n*-hexadecyl, *n*-heptadecyl and *n*-octadecyl; preferred meanings of -Z- are a single covalent bond, or a group of formula -O-, or -NHC(=O)O-, and still more preferred are a group of formula -O- or -NHC(=O)O-; a preferred meaning of -Y- is a group $-C(=O)NR_2$-, where $R_2$ is H, $-C(=O)CH_3$ or -C(=O)-OEt, and still more preferably $R_2$ is a group $-C(=O)CH_3$ or -C(=O)OEt, and still even more preferably $R_2$ is a group $-C(=O)CH_3$; preferred values for n are 1 to 8, and still more preferred are 1 or 4 to 7, and still more preferred is 1; preferred values for p and m are p=m=0, or p=1, m=2-6, and still more preferred p=m=0; preferred examples of the Q radical are 3-thiazolium, 2-pyridyl, and 1-ethyl-2-pyridinium, and still more preferred is 1-ethyl-2-pyridinium; preferred anions $A^-$ are *p*-toluenesulfonate, iodide and chloride, and still more preferred are iodide and chloride, and even more preferred is chloride; q is preferably 1.

Furthermore, among the compounds of formula I there are still ferred the specific compounds whose formulas are represented below, together with the number corresponding to the example in which their preparation is described:

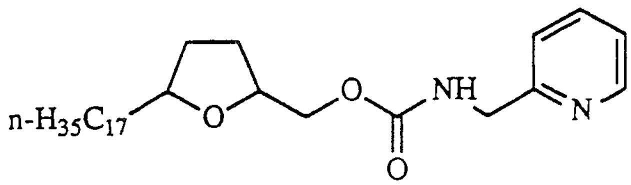

1

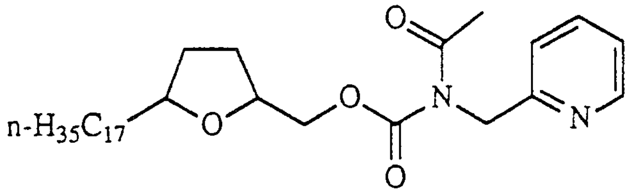

2

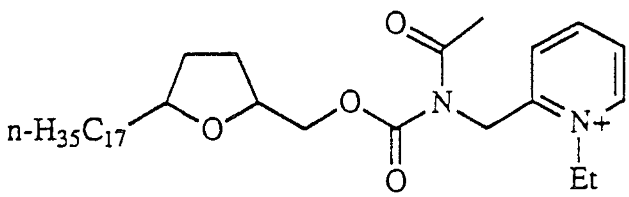

.I-

3

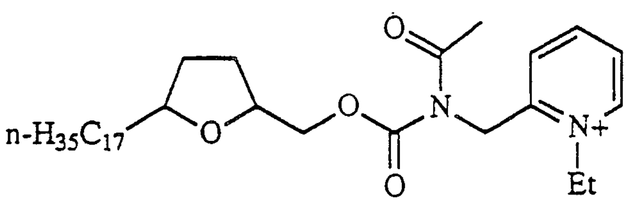

. Cl-

4

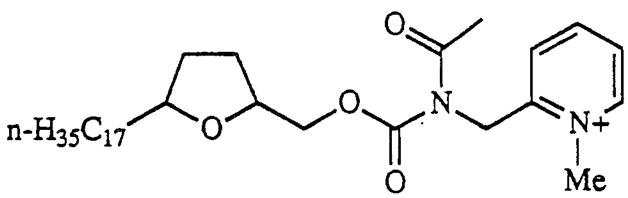

.I-

5

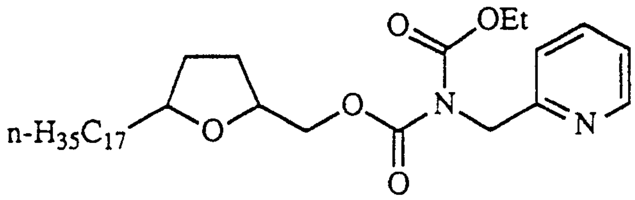

6

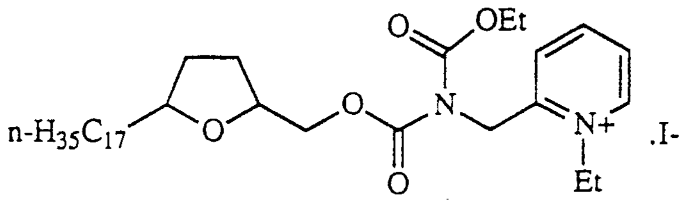

7

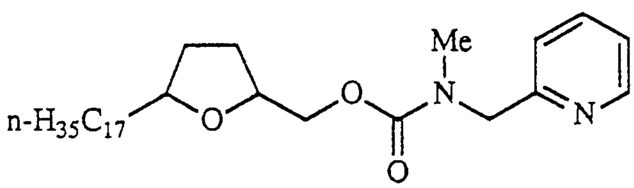

8

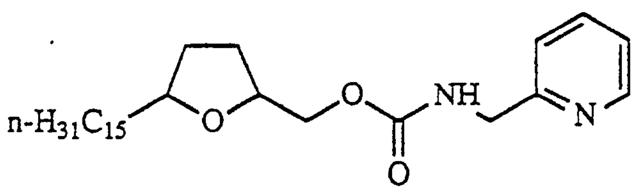

9

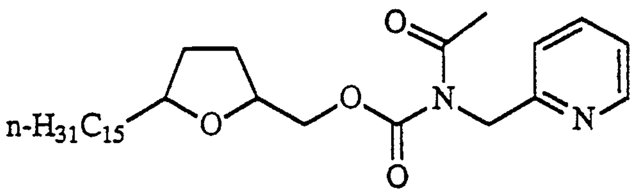

10

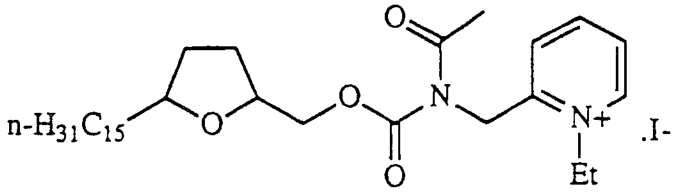

11

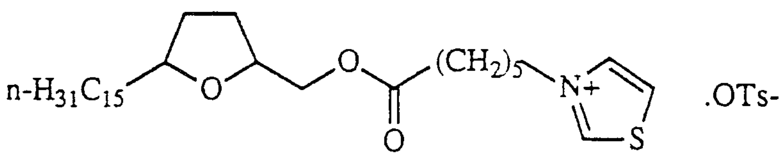

12

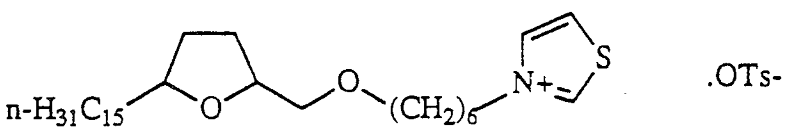

13

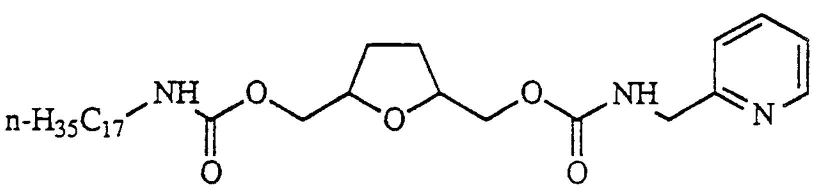

14

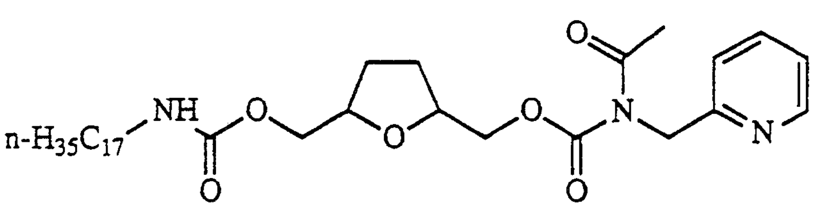

15

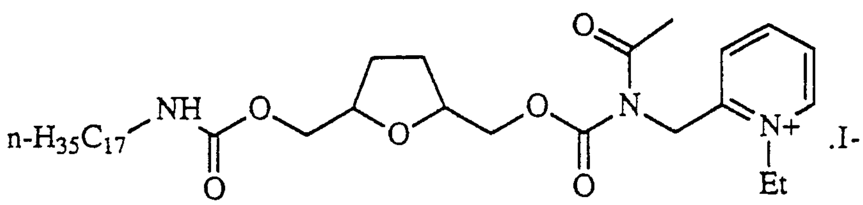

16

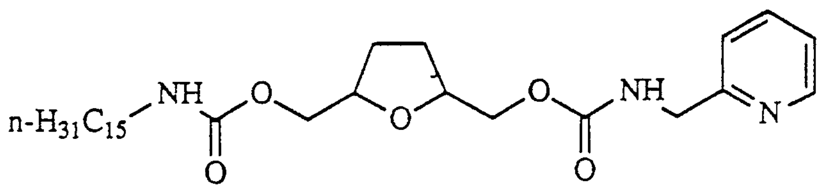

17

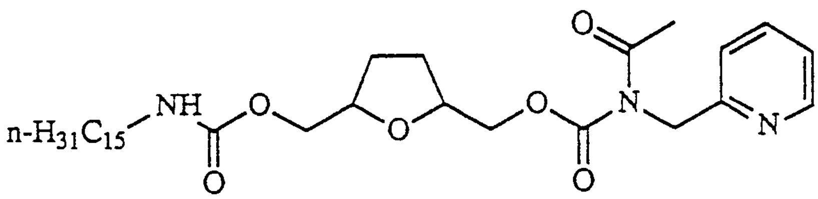

18

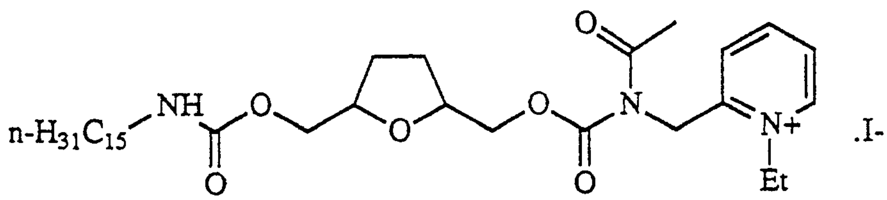

19

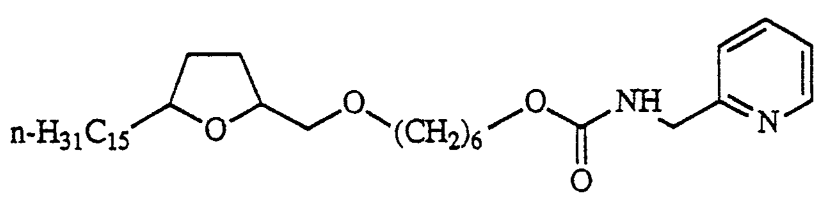

20

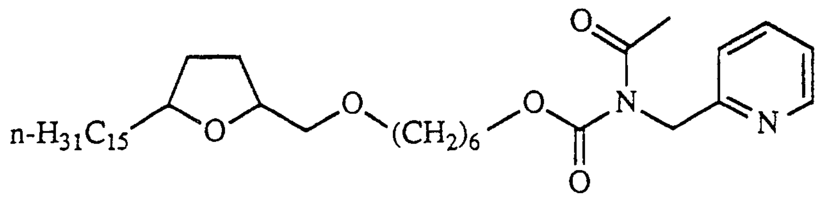

21

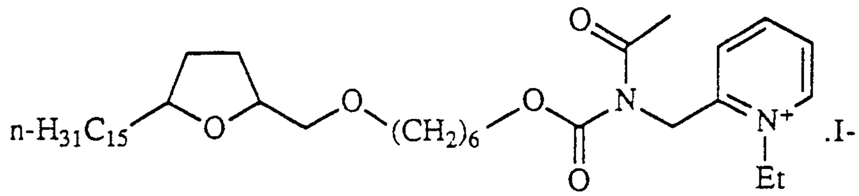

22

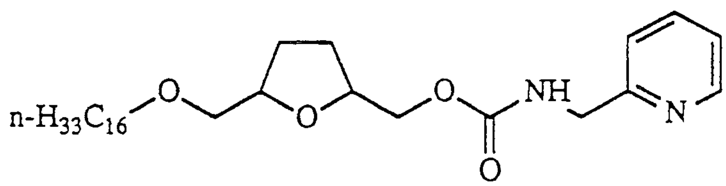

23

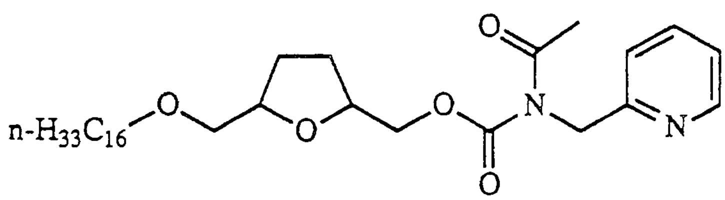

24

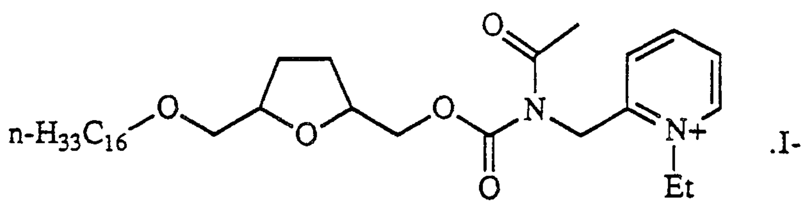

25

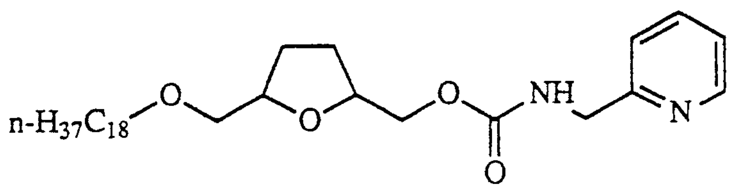

**26**

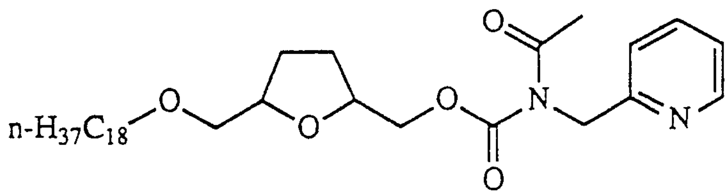

**27**

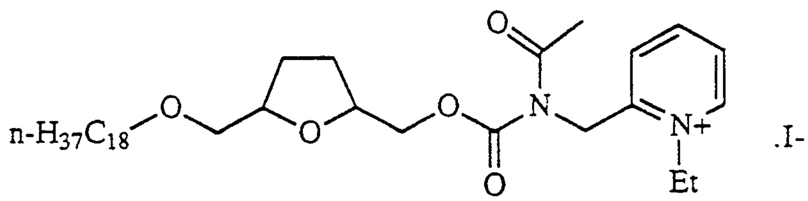

**28**

The compounds of the present invention are *in vitro* inhibitors of the platelet aggregation induced by PAF. On the other hand, these compounds have the capacity to revert the hypotension induced by PAF in anesthetized rats. These facts make them useful as a PAF antagonists in the treatment of the diseases in which this substance is involved.

Since the compounds of formula **I** exhibit excellent PAF antagonism, they are therefore useful as prophylactic and therapeutic agents of circulatory disturbances due to PAF, for example, thrombosis, apoplexy (eg, cerebral hemorrhage, cerebral thrombosis), myocardial infarction, angina pectoris, venus thrombosis, nephritis (eg, glomerular nephritis), diabetic nephritides, shock (eg, endotoxin shock observed after grave infectious diseases or surgical operation, intravascular hemagglutination syndrome caused by endotoxin, anaphylactic shock, hemorrhagic shock); gastroenteric diseases caused by PAF (eg, gastric ulcer); diseases associated with allergy and inflammation (eg, bronchial asthma, psoriasis); pneumonia; rejection symptoms associated with increase in the amount of PAF produced in the case of internal organ transplantation; insufficiency of internal organ (eg, heart, liver, kidney) in the case of internal organ operation, etc. The compounds of formula **I** can be used also for the pourpose of contraception of female mammals bi inhibiting cytodieresis and/or implantation to uterus.

A further purpose of the present invention is to provide a process for the preparation of the compounds having the formula **I** starting from a compound of formula **II:**

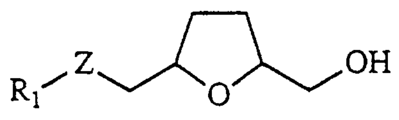

II

Compounds of formula **II** in which z is a covalent single bond, can be prepared following a slightly modified procedure described in the literature (*J.Med.Chem.* .**1982**, 25, 121-125) as shown in scheme I. The first step (step A) is done following the published method. The cyclation step (step B) involves two stages that are done in the same reaction medium, that is the epoxidation of the double bond and the cyclation *in situ* to the corresponding compound **II**. As a epoxidating agent the election reagent is *m*-chloroperbenzoic acid. There is no particular restriction on the nature of the solvent to be employed, provided that it does not interfere with other parts of the molecule and that it can dissolve the starting materials at least to some extent. Examples of suitable solvents include dichloromethane and chloroform. The reaction will take place over a wide range of temperatures and the precise temperature chosen is not particularly critical. We generally find it convenient to conduct the reaction at a temperature from 0 °C to that of the boiling solvent, preferably at room temperature. The time required for the reaction may vary widely, depending on many factors, notably the reaction temperature and the nature of the starting materials employed, but a period of from 1h to 48 h will normally suffice. We have observed that, some times, a mixture of the desired product and the intermediate epoxide is obtained. In that case, the reaction can be driven to completion by addition of a catalytic amount of acid such as percloric or trifluoroacetic acids. The desired product can be then purified by chromatography or recrystallization.

## Scheme I

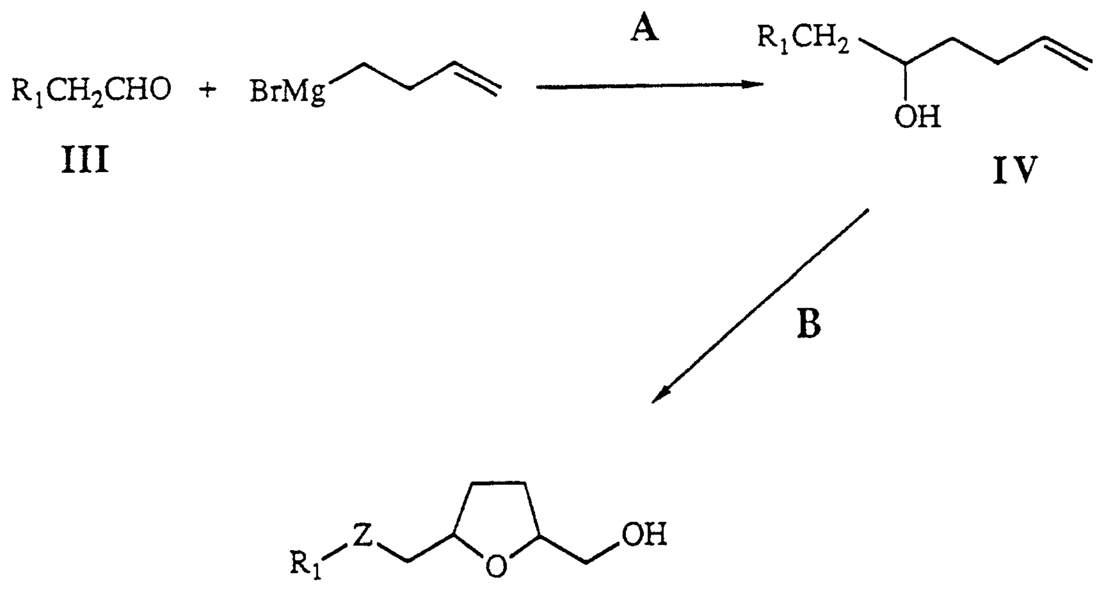


III

IV

II (Z = single bond)

Compounds of formula **II** in which Z is a O, C(=O)O, OC(=O)O or $NR_2C(=O)O$ group, can be prepared according to scheme II, in which P is a suitable hydroxyl protecting group. According to this scheme, steps A and B are the same as the above mentioned for the scheme I. Step C involves the reaction of alcohol **VII** with a W-$R_1$ compound, where W is a O=C=N-, ClC(=O)-, ClC(=O)O- group, or a leaving group such as halogen (Cl, Br, I) or aryl or alkylsulfonate ($CH_3SO_3$-, p-$CH_3C_6H_4SO_3$). When W is O=C=N-. ClC(=O)- or ClC(=O)O- then, Z is, respectively, -NHC(=O)O-, -C(=O)O- or -OC(=O)O- and the corresponding step C is usually done in a solvent the nature of which is not critical, provided that it does not interfere with other parts of the molecule and that it can dissolve the starting materials at least to some extent. Examples of suitable solvents include dichloromethane and chloroform, ethers such as diethylether, tetrahydrofuran or dioxane, and aromatic hydrocarbons such as benzene or toluene. The reaction is carried out in the presence of a base, that can be used as a solvent. Although there is no restriction about the base used, tertiary amines such as triethylamine or pyridine are preferred. The reaction will take place over a wide range of temperatures and the precise temperature chosen is not particularly critical. We generally find it convenient to conduct the reaction at a temperature from 0° to 100° C, but temperatures from 0° to 50° C are preferred. The time required for the reaction may vary widely, depending on many factors, notably the reaction temperature and the nature of the starting materials employed, but a period of from 30 min to 24 h is usually satisfactory. Although the reaction is clean, the product **VIII** if desired, can be purified by flash chromatography.

When Z is an oxygen atom, step C is done using a reagent W-$R_1$, where W is a leaving group, and the previously prepared alkoxide of compound **VII**. This preparation is carried out by treatment of compound **VII** with a strong base, such as sodium hydride or *n*-butyllithium, in a solvent that does not interfere with the reagents. Examples of suitable solvents include dimethylformamide or tetrahydrofuran. The alkoxide formation is carried out between 0 and 80° during a period of time between 10 min and 2 h. This is followed by the addition of the reagent W-$R_1$ which is allowed to react at a similar temperature margin,

during a period of time of from 1 and 24 h, depending on the nature of W and the temperature used. The desired compound is isolated by conventional methods. If desired, it can be purified by flash chromatography.

The last step (D) of scheme II involves the deprotection of the hydroxyl function to afford compound **II**. The reagents and conditions used in such a transformation depend, of course, on the nature of the protecting group P, which can be any of the usual hydroxyl protecting groups, such as benzyl, trityl, tetrahydropyranyl, trialkylsilyl etc. We have found that the benzyl group is particularly convenient in the present synthesis. Reaction D means, in this case, a reduction. Such a transformation can be carried out in a catalytic way at room temperature, using palladium over carbon, or platinum, as a catalyst in the presence of hydrogen gas. The reaction is carried out preferentially in a solvent, provided that it does not interfere with other parts of the molecule and

## SCHEME II

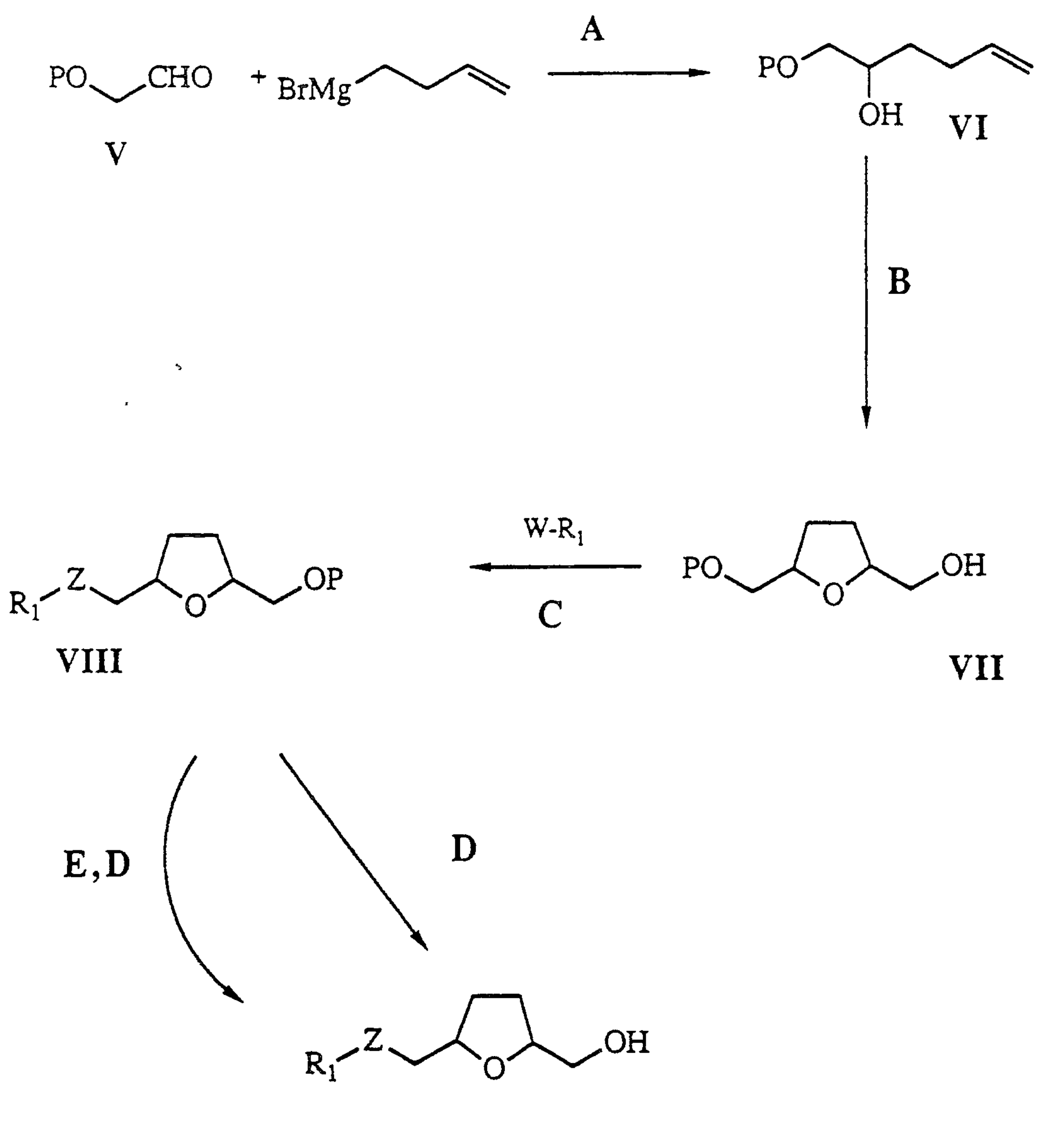


**II** (Z = -O-, -C(=O)O-, $-NR_2C(=O)O-$)

that it can dissolve the starting materials at least to some extent. Examples of suitable solvents include

alcohols, such as methanol or ethanol; ethers such as tetrahydrofuran or dioxane; organic acids such as acetic acid; and mixtures of one or more of these solvents with water. The required reaction time will vary according to the nature of the substrate, the amount of catalyst and the pressure of hydrogen in the vessel. Usually, a period of from 5 min to 24 h will normally suffice. After completion of the reaction, the desired compound can be isolated by simple filtration and solvent evaporation. The product obtained in this way is usually pure enough to be used in the next step.

In the case where in compound **II** the Z group is $-NR_2C(=O)O-$with $R_2 \neq H$, then compound **VIII** must be *N*-alkylated or *N*-acylated previously to the deprotection of the hydroxyl group. This reaction (step E) is done in two stages, that is, formation of the amide anion and reaction of this amide with the alkylating or acylating agent $X-R_2$, wherein X is a leaving group such as a halogen atom (Cl, Br or I) or an alkyl or an arylsulfonate group ($CH_3SO_3-$, $p-CH_3C_6H_4SO_3-$). The formation of the amide anion of compound **VIII** ($Z=-NHC(=O)O-$) is carried out using a strong base such as n-butyllithium. There is no particular restriction on the nature of the solvent to be employed, provided that it does not interfere with other parts of the molecule and that it can dissolve the starting materials at least to some extent. Examples of suitable solvents include dimethylformamide and tetrahydrofuran. The reaction will take place over a wide range of temperatures and the precise temperature chosen is not particularly critical. We generally find it convenient to conduct the reaction at a temperature from -75 and 0°. The time required for the reaction may vary widely, depending on many factors, notably the reaction temperature and the nature of the starting materials employed, but a period of from 5 min to 3h is usually enough. In the second stage of step E, the amide anion formed is allowed to react *in situ* with the reagent $X-R_2$. The reaction can be done between broad temperature margins but usually it is convenient to perform it between -50° and 0°. The required reaction time depends notably on the nature of the reagent $X-R_2$ and the temperature used. Usually, a period of time of from 10 min to 24 h will suffice. After completion of the reaction, the desired product can be isolated and purified by conventional methods. The product obtained in this way (**VIII**, $Z=-NR_2C(=O)O-$) is converted into the corresponding compound **II** ($Z=-NR_2C(=O)O-$) following the methodology described for the step D.

Alternatively, compounds of formula **II** in which 2= -O-, -C(=O)O-, -OC(=O)O- or $-NR_2C(=O)O-$, can be prepared in a similar way to that described in scheme II but using the compound **IX** as a starting material, according to scheme III. The compound **IX** can be obtained according to the literature (*cis: Tetrahedron*, **1965**, 21, 2353-2358; ***trans:*** *J.Chem. Soc.*, **1900**, 103).

## SCHEME III

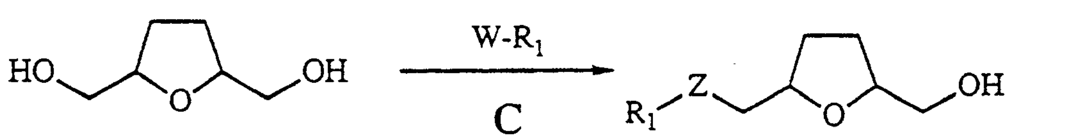


IX

II (Z = -O-, -C(=O)O-, $-NR_2C(=O)O-$)

Scheme IV shows the synthesis of the compounds of subfamilies **Ia** and **Ib** that is, compounds of formula **I** where Y is $-C(=O)NR_2-$ with $R_2$ different or equal to H respectively, m=p=0, and $Q^{(+)}$ is an heterocycle that contains a quaternary nitrogen and that is linked to the alkylene chain by a ring carbon. According to that scheme, in step F alcohol **II** is treated with a compound **X** which is one of the so-called phosgene equivalents, that is a doubly activated carbonyl group. In compound **X** the groups X and X′ are leaving groups and can be equal (Cl, imidazole, etc) or different. Although, in principle, any phosgene equivalent described in the literature could be used, we have found that phenyl chlorocarbonate (**X**, X=Cl, X′=OPh) is an excellent reagent to carry out this reaction, due to its convenience to handle and its low cost. The reaction is carried out in a solvent and in the presence of a proton scavenger base. There is no particular restriction on the nature of the solvent to be employed, provided that it does not interfere with other parts of the molecule and that it can dissolve the starting materials at least to some

## SCHEME IV

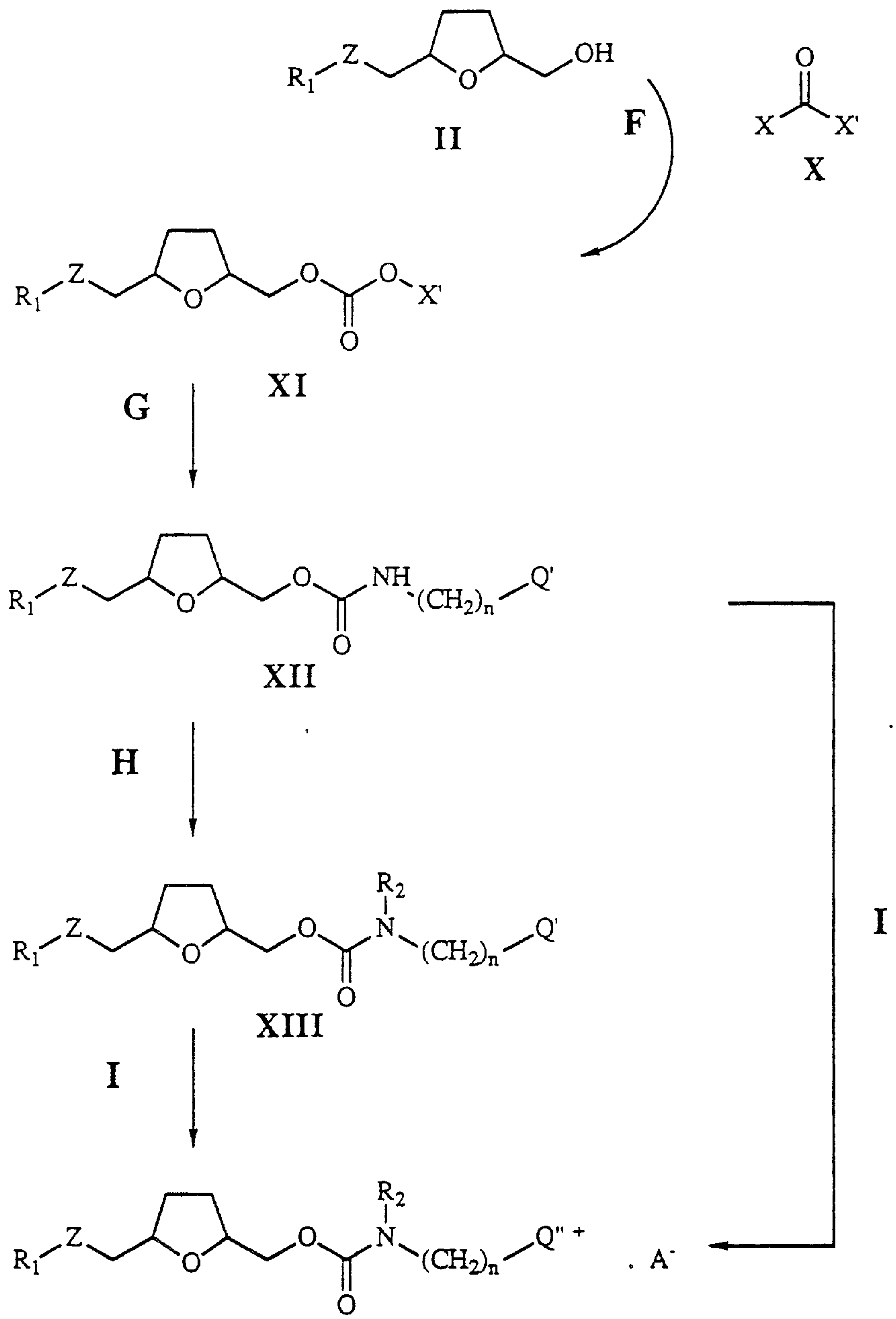


II

X

XI

XII

XIII

**Ia** ($R_2$=H)

**Ib** ($R_2$=H)

extent. Examples of suitable solvents include halogenated hydrocarbons, particularly, dichloromethane and chloroform; ethers like diethylether, tetrahydrofuran or dioxane; and aromatic hydrocarbons like benzene or toluene. Neither any particular restriction exist with respect to the base, provided that it does not interfere with other parts of the molecule. It is preferred to use an amine such as triethylamine or pyridine. The reaction will take place over a wide range of temperatures and the precise temperature chosen is not particularly critical. We generally find it convenient to conduct the reaction at a temperature from 0 to 100 °C, but temperatures from 0 to 50 ° are preferred. The time required for the reaction may vary widely, depending on many factors, notably the reaction temperature and the nature of the starting materials employed, but a period of from 30 min to 24 h is usually satisfactory. The reaction is clean and usually a purification of the product is not necessary. However, if desired, the product **XI** can be purified by flash chromatography.

In step G, compound **XI** (obtained in step F) is converted into the carbamate **XII**. by reaction with an amine of formula $H_2N\text{-}(CH_2)n\text{-}Q'$ in which n is an integer from 0 to 10 and Q′ is an heterocyclic radical that contains a non-quaternized nitrogen atom that is linked to the alkylene chain by a ring carbon; Q′ can also be an OP group where P is an oxygen protecting group. The reaction is done in a solvent, at a temperature and a period of time similar to those described in the step F. The reaction crude is washed, in this case, with an alkaline aqueous solution in order to take off the phenol produced during the reaction. The product obtained (**XII**) can be purified by conventional methods such as flash chromatography.

When Q′ = OP, the conversion of product **XII** into the product of formula **I** in which Y is a $\text{-CONR}_2\text{-}$ group and Q is a radical $Q''^{(+)}$, takes place by means of the steps M,N and K that will be described in the discussion of scheme VI.

When Q′ is an heterocycle that contains a non-quaternized nitrogen atom and that is linked to the alkylene chain by a ring carbon, the conversion of compound **XII** into the product **Ia** is done by steps H and I that will be explained next.

Step H involves the derivatization of the carbamic nitrogen of compound **XII** to give a compound **XIII**. The reaction involves the use of a reagent of formula $R_2\text{-}X$ where $R_2$ and X have the same meaning as above. In the case in which $R_2$ is acyl, a compound of formula $(R_2)_2O$ can also be used. The reaction can be carried out in two ways: directly using compound **XII** or preparing first an alkaline salt of it. For $R_2$ equal to acyl or alkoxycarbonyl both methods can be used whereas for $R_2$ being alkyl, the second method is necessary. When compound **XII** is used directly, the reaction is carried out in a solvent that it does not interfere with other parts of the molecule and that it can dissolve the starting materials at least to some extent. We have found that the halogenated hydrocarbon solvents like dichloromethane or chloroform are efficient in this reaction. On the other hand, the reaction can be carried out in the presence of a proton scavenger amine like triethylamine. The use of the amine is not compulsory because the heterocycle Q′ already has a basic nitrogen. The reaction is done in a broad range of temperatures, from 0 ° to the boiling point of the solvent, but a temperature near room temperature is usually preferred. The time required for the reaction may vary widely, depending on many factors, notably the reaction temperature and the nature of the starting materials employed, but a period of from 2h to 72 h is usually enough. After completion of the reaction, the desired product, of formula **XIII** can be isolated and purified by conventional methods such as flash chromatography.

In the case in which an alkaline salt of compound **XII** is used, this salt is formed in the same reaction medium using a strong base like sodium hydride or *n*-butyllithium. To the same reaction medium is next added compound $R_2X$. The reaction is preferently carried out in the presence of a solvent the nature of which is not critical, provided that it does not interfere with other parts of the molecule and that it can dissolve the starting materials at least to some extent. As examples of preferred solvents, ethers like diethylether, tetrahydrofuran or dioxane and aromatic hydrocarbons like benzene or toluene, can be mentioned. The reaction can be carried out at a broad range of temperatures, however we have found that it is better to do it at lower temperatures, for instance between -78 ° and 0 °C if an acceptable yield is desired. The reaction will take place over a wide range of temperatures and the precise temperature chosen is not particularly critical. We generally find it convenient to conduct the reaction at a temperature from 5 min to 2 h. The desired product can be isolated and purified by conventional methods.

Step I„ involves the transformation of a compound **XIII** or **XII** in a compound **Ia** or **Ib** respectively, wherein $Q^{(+)}$ represents an heterocyclic radical that contains a quaternary nitrogen in the ring and that is linked to the alkylene chain by a carbon, and $A^-$ is a pharmaceutically acceptable anion. The reaction takes place between the starting material and a reagent of formula $R_3A$ wherein $R_3$ is a lower alkyl group. The reaction can be done without solvent in the case that $R_3A$ is liquid and not very volatile or in the presence of a solvent when $R_3A$ is solid or very volatile, but in any case an excess of reagent is always used. Examples of suitable solvents include those with high polarity. As examples of preferred solvents, we can

mention acetonitrile, tetrahydrofuran, dioxane, dimethylformamide or dimethylsulfoxide. The reaction will take place over a wide range of temperatures and the precise temperature chosen is not particularly critical. The time required for the reaction may vary widely, depending on many factors, notably the nature of the reagent $R_3A$ and the temperature used, but a period of time between 1h and 72 h will generally suffice. The desired compound is isolated by concentration of the crude reaction or by precipitation with a less polar solvent. The compound obtained in that way is usually pure enough. If that is not the case, it can be purified by conventional techniques such as flash chromatography or recrystallization.

Compounds of formula **Ia** or **Ib** are salts wherein the anion A-comes from the reagent $R_3A$. If desired, such anion can be changed by using an ionic interchange resin.

Scheme V shows the synthetic steps that give rise the products of the subfamily **Ic**, that is the compounds of formula **I** in which Y is a -C( = O)- group, m = p = 0 and Q is an heterocyclic radical linked to the alkylene chain directly by the quaternary ring nitrogen (in that case Q is represented by Q‴).

Step J involves the reaction of **II** with a compound of formula $ClC(=O)-(CH_2)_n-A$ to give **XVI** wherein A is a leaving group such as an halogen atom or an alkyl or arylsulfonate group. The reaction conditions like temperature or reaction time, as well as the isolating methods and purification of the final products are similar to those described for step F.

In step K the compound **XVI** is treated with an heterocycle that contains at least a tertiary nitrogen in the ring, to give **Ic**, where Q‴ is an heterocyclic radical linked to the alkylene chain by the quaternary

## SCHEME V

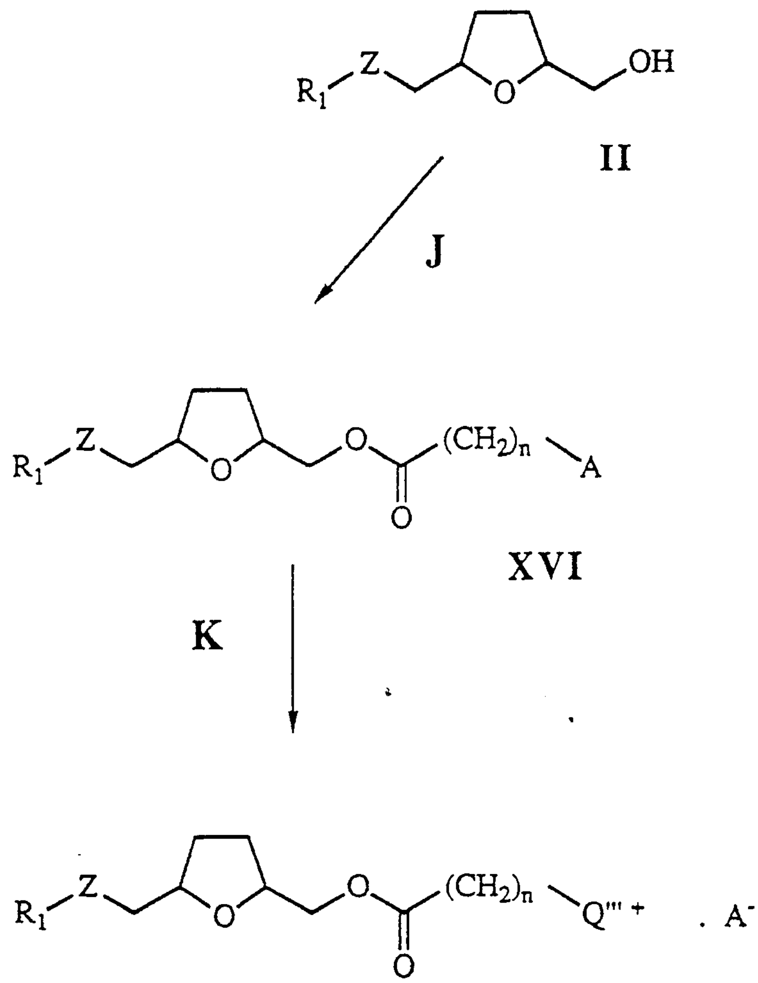


II

XVI

Ic

nitrogen of the ring. The reaction can be done using the heterocycle itself as a solvent or adding a cosolvent, preferentially of polar character, such as acetonitrile, tetrahydrofuran, dimethylformamide or dimethylsulfoxide. The reaction will take place over a wide range of temperatures and the precise temperature chosen is not particularly critical. We generally find it convenient to conduct the reaction at a temperature from 25 to 120 °C, and preferentially from 50 to 90 °C is. The time required for the reaction may vary widely, depending on many factors, notably the reaction temperature and the nature of the starting materials employed, but a period of from 1 h to 48 h is usually enough. The desired compound can be isolated by evaporation of the volatile components and precipitation with a slightly polar solvent like ether. If desired, the product can be purified by one or several recrystallizations.

Scheme VI shows the synthesis of compounds of the subfamily **Id**, that is, compounds of formula **I** in which Y is a covalent single bond, m = p = 0 and the heterocyclic radical Q is linked to the alkylene chain directly by the quaternary nitrogen of the ring (in which case Q is represented by Q>). In the first step (step L), alcohol **II** is converted to the corresponding alkoxide and is treated with a compound of formula A-(CH2)-

$_n$-OP in which A is a leaving group, such as a halogen atom or alkyl or arylsulfonate group, and P is a hydroxyl protecting group. The reaction has two stages, *ie* the formation of the alkoxide and the reaction with the alkylating agent, both of them carried out in the same reaction media. For the alkoxide formation, a strong base like sodium hydride or *n*-butyllithium can be used. In the second stage, the addition of the alkylating agent is generally done in the same solvent that is used in the reaction. That solvent can be, in principle, any polar solvent provided that it does not interfere with other parts of the molecule and that it can dissolve the starting materials at least to some extent. An example of a suitable solvent is dimethylformamide. The reaction will take place over a wide range of temperatures and the precise temperature chosen is not particularly critical. We generally find it convenient to conduct the reaction at a temperature from 50 to 120 °C. The time required for the reaction may vary widely, depending on many factors, notably the reaction temperature and the nature of the starting materials employed, but a period of from 4 to 30 h will generally suffice. After completion of the reaction, the desired product is isolated by conventional methods. If desired, the product can be purified by flash chromatography.

Step M involves the deprotection of the hydroxyl group of compound **XVIII**. The reagents and conditions used depend mainly on the nature of the P group used. P can be any of the protecting groups of the hydroxyl function commonly used such as benzyl, trityl, trialkylsilyl or tetrahydropyranyl. In the present invention we have used a

## SCHEME VI

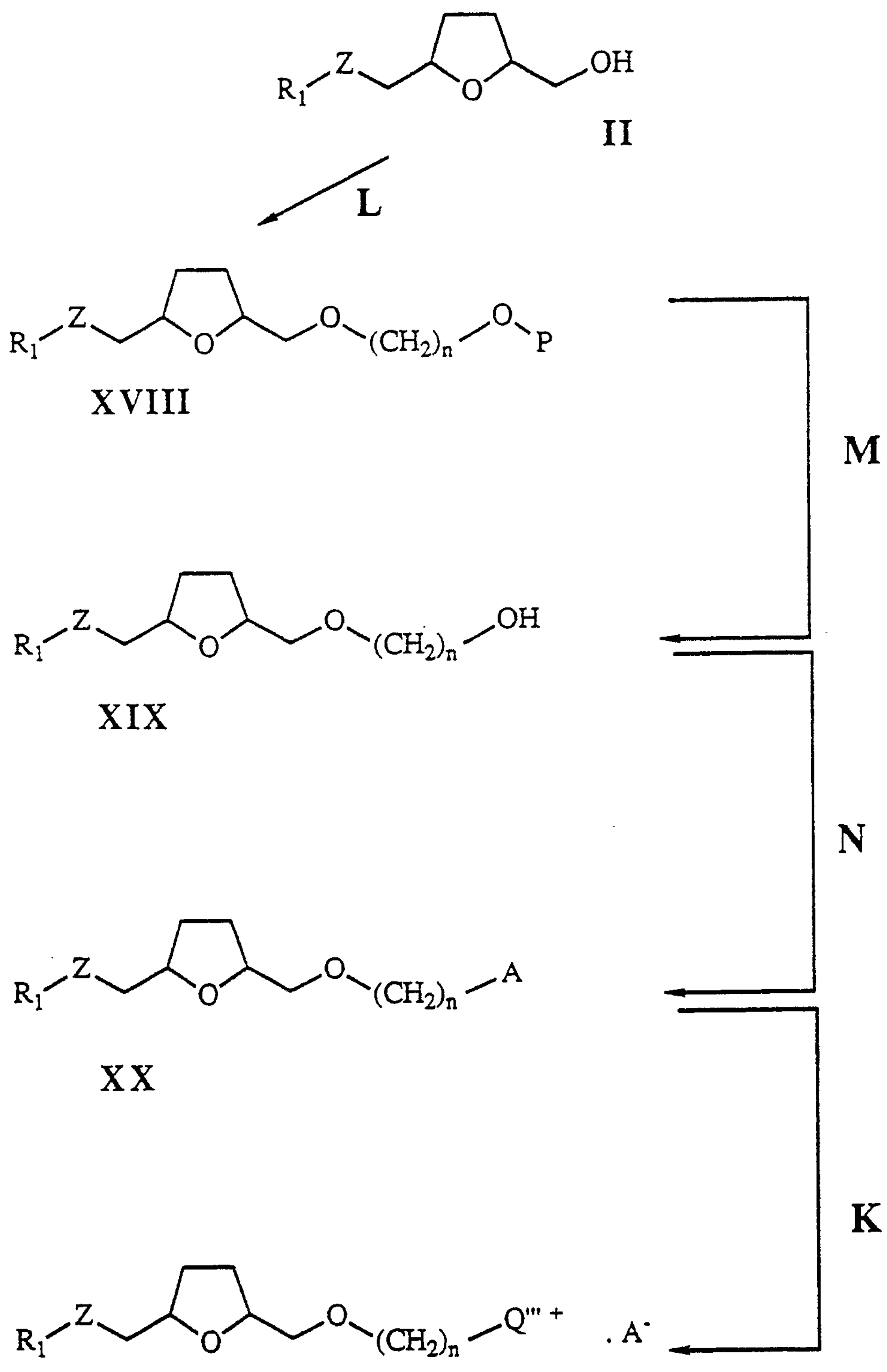


Id

tetrahydropyranyl group so step M involves a hydrolysis or acid alcoholysis reaction. The reaction can be done in a solvent that, in the case of the alcoholysis will be the alcohol itself, for instance methanol, and in the case of the hydrolysis will be a mixture of water and a cosolvent, for instance tetrahydrofuran. In both cases an acid has to be used as a catalyst. Such acid can be, in principle, any acid relatively strong, such as chlorhydric, sulfuric, camphorsulphonic or toluensulphonic. The reaction will take place over a wide range of temperatures and the precise temperature chosen is not particularly critical. We generally find it convenient to conduct the reaction at a temperature from 0 to 50 °C. The time required for the reaction may vary widely, depending on many factors, notably the reaction temperature and the nature of the starting materials employed, but a period of from from 1 to 12 h will generally suffice. After completion of the reaction, the medium is neutralized and the desired product is isolated and purified by conventional methods.

Step N involves the conversion of the hydroxyl group of compound **XIX** into a leaving group A. In the case that A is an alkyl or arylsulfonate group, compound **XX** is obtained by reaction of **XIX** with the corresponding alkyl or arylsulfonate chloride in the presence of a base. The nature of the solvent and base employed, as well as the conditions of temperature and reaction time used are similar to those described in step E.

Finally, compound **XX** can be converted into the final product **Id** by means of the methodology described in step K.

Scheme VII shows the synthesis of the compounds of subfamily **Ie**, that is, the compounds in which Y is $-C(=O)NR_2-$, p = 1, m is not 0 and $Q''(+)$ is an heterocycle that contains a quaternary nitrogen, linked to the alkylene chain by a ring carbon. The preparation of such compounds is carried out starting from alcohol **XIX** of scheme VI in which n has been substituted by m. Such synthesis is carried out in the same way as described in scheme IV, *ie*, by means of steps F,G,H and I.

## SCHEME VII

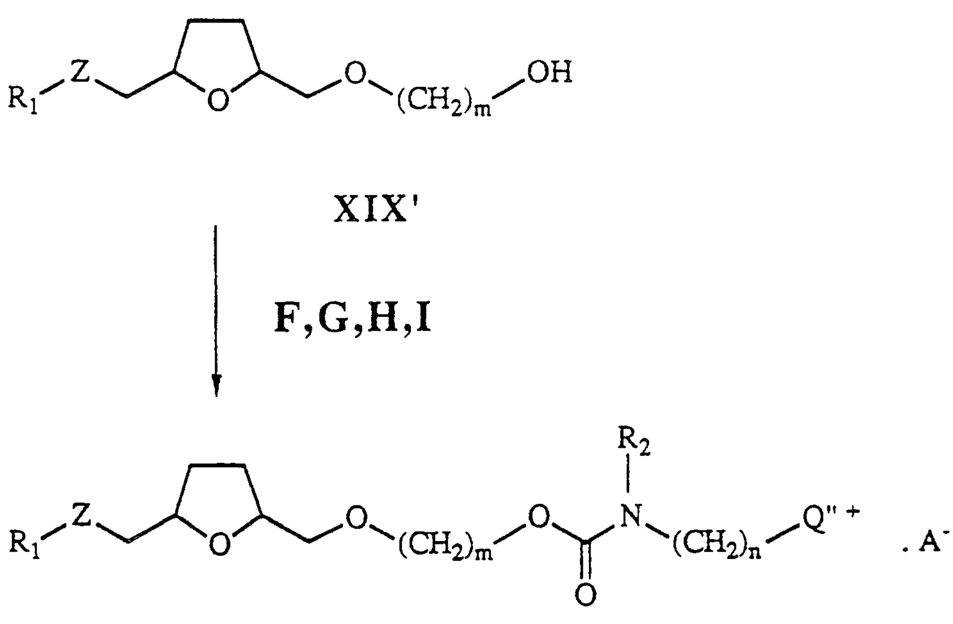


XIX'

Ie

Compounds of general formula **I** are useful as PAF inhibitors, as demonstrated by their ability to inhibit the *in vitro* platelet aggregation induced by PAF in rabbit according to test 1:

Test 1: Inhibition of platelet aggregation induced by PAF.

The blood is obtained by cardiac puncture of male New Zealand albino rabbits (between 2 and 2.5 Kg of weight) and coagulation is prevented by adding 1 part of 3.16% sodium citrate dihydrate in 9 parts of blood. The platelet rich plasma (PRP) is prepared by blood centrifugation at 250xg for 10 min. at 4° C and it is diluted with platelet poor plasma (PPP) obtained by additional centrifugation at 3000xg for 10 min. The amount of platelets is adjusted to $3x10^{-5}/mm^3$. The platelet aggregation induced by PAF ($C_{18}$, prepared in our laboratory) (16 nM, final) is determined by the Born nephelometric technique (*J. Physiol.*, **1962** 162, 67) using a aggregometer Chrono-log 500. The activities of the inhibitors are expressed as $IC_{50}$, that is to say the concentration of the drug needed to inhibit the platelet aggregation in a 50%. The results are shown in table 1:

Table 1.

| Compound number | $IC_{50}$(μM) |
|---|---|
| 3 | 0.071 |
| 4 | 0.19 |
| 5 | 1.1 |
| 7 | 0.13 |
| 11 | 0,095 |
| 12 | 3,1 |
| 13 | 1,3 |
| 16 | 0,04 |
| 19 | 0.054 |
| 25 | 0.074 |

Furthermore, it has been found that the title compounds I are inhibitors of the hypotension induced by PAF according to test 2.

Test 2 - Inhibition of the hypotensive effect induced by PAF in normotense rats.

Male Sprage Dawley rats, of 180-220 g of weight, anesthetized with sodium pentobarbital (50 mg/Kg, i.p. 1 mL 100 g) have been used. In order to measure the average arterial pressure, a polyethylene catheter is introduced into the carotid artery. The arterial pressure is recorded with the help of a transducer connected with a R611 Beckman polygraph. The tested compounds are administrated through the femoral vein 3 min. before the injection of PAF (0.5 mcg/Kg, i.v.) The inhibition of the hypotension induced by PAF of the different compounds expressed as $IC_{50}$, is shown in table 2.

Table 2.

| Compound number | $CI_{50}$ (mg/Kg,i.v.) |
|---|---|
| 3 | 0,15 |
| 4 | 0,19 |
| 5 | 0,8 |
| 7 | 0,34 |
| 11 | 0,15 |
| 12 | 1,2 |
| 13 | 2 |
| 16 | 0.064 |
| 19 | 0.098 |
| 25 | 0.036 |

Solid compositions according to the present invention for oral administration include compressed tablets. dispersible powders. granules and capsules. In tablets. one or more of the active component(s) is admixed with at least one inert diluent such as lactose. starch. mannitol. microcrystalline cellulose or calcium phosphate; granulating and desintegrating agents for example corn starch. gelatine, microcrystalline cellulose or polyvinylpyrrolidone; and lubricating agents for example magnesium stearate, stearic acid or talc. The tablets may be coated by known techniques to delay disintegration and absorption in the gastrointestinal tract and, thereby, provide a sustained action over a longer period. Gastric film-coated or enteric film-coated can be made with sugar, gelatin, hydroxypropylcellulose, or acrylic resins. Tablets with a sustained action may also be obtained using an excipient which provides regressive osmosis, such as the galacturonic acid polymers. Formulations for oral use may also be presented as hard capsules of absorbable material, such as gelatin, wherein the active ingredient is mixed with an inert solid diluent and lubricating agents, or pasty materials, such as etoxylated saturated glycerides. Soft gelatin capsules are possible wherein the active ingredient is mixed with water or an oil medium, for example peanut oil, liquid paraffin or olive oil.

Dispersible powders and granules suitable for preparation of a suspension by the addition of water provide the active ingredient in admixture with a dispersing or wetting agent, a suspending agent, such as sodium carboxymethylcellulose, sodium alginate. polyvinylpirrolidone, gum tragacanth, xantham gum, gum acacia. and one or more preservatives, such as methyl or *n*-propyl-*p*-hydroxybenzoate. Additional excipients. for example sweetening, flavoring and coloring agents may also be present.

Liquid compositions for oral administration include emulsions, solutions, suspensions, syrups and elixirs containing commonly used inert diluents, such as distilled water, ethanol, sorbitol, glycerol, or propylene glycol. Such compositions may also comprise adjuvants such as wetting agents, suspending agents, sweetening, flavoring, perfuming, preserving agents and buffers.

Other compositions for oral administration include spray compositions, which may be prepared by known methods and which comprise one or more active compound(s). The spray compositions will contain a suitable propellent.

Preparations for injection according to the present invention for parenteral administration include sterile aqueous or non-aqueous solutions, suspensions or emulsions, in a non-toxic parentally-acceptable diluent or solvent. Examples of aqueous solvents or suspending media are distilled water for injection, the Ringer's solution, and isotonic sodium chloride solution. Examples of non-aqueous solvents or suspending media are propylene glycol, polyethylene glycol, vegetable oils such as olive oil, or alcohols such as ethanol. These compositions may also include adjuvants such as wetting, preserving, emulsifying and dispersing agents. They may be sterilized by one of the known methods or manufactured in the form of sterile solid compositions which can be dissolved in sterile water or some other sterile injectable medium immediately before use. When all of the components are sterile, the injectables will maintain the sterility if they are manufactured in sterile environment.

A compound of the invention may also administered in the form of suppositories for rectal administration of the drug, or as creams, ointments jellies, solutions or suspensions for topical use and pessaries for vaginal administration.

The compounds of formula I are low in toxicity, and can therefore be administered orally or non-orally as it is in a form of powder, or as one of the above-mentioned pharmaceutical compositions in a suitable

dosage form, to mammals (eg, man, rabbit, dog, cat, rat, mouse and guinea pig). The dosage varies depending upon the subject to be administered, disease to be treated, conditions thereof and route of administration, and when the compounds of formula I are used for prophylaxis or therapy of shock in a human adult, it is convenient to administer through intravenous injection usually in a single dose in the range of from 0.001 to 1.0 mg/kg body weight, preferably from about 0.01 to 0.1 mg/kg body weight, about once to five times a day, preferably about once to three times a day. And the compounds of formula I can also be administered trhough drip injection in a single dose in the range of about from 0.01 to about 0.1 mg/kg body weight/min. for about one hour, about once to five times a day, preferably once to three times a day. The dosages for other non-oral routes as well as the oral dosage may be selected referring to above-mentioned dose levels. When shock symptoms are very serious, dosage may be increased depending on the symptoms.

When the compounds of formula I are used orally for the prophylaxis or trherapy of, for example, thrombosis, bronchial asthma, nephritis etc, in a human adult, it is convenient to administer usually in a single dose in the range of from about 0.1 to 30 mg/kg body weight, preferably in the range of from 1 to 10 mg/kg body weight, about once to five times a day, preferably from once to three times. The dosages for the non-oral routes may be selected referring to the above-mentioned dose levels.

The pharmaceutical composition to be used for the above administration comprises an effective amount of the compound of formula I and a pharmaceutically acceptable carrier or excipient and the said composition is provided in a dosage form suitable for oral or non-oral administration.

Following are some representative preparations for tablets capsules, syrups, aerosols and injectables. They can be prepared following standard procedures and they are useful as inhibitors of the platelet activating factor.

## Tablets

| | | |
|---|---|---|
| Title compound I | 100 | mg |
| Dibasic calcium phosphate | 125 | mg |
| Sodium starch glycolate | 10 | mg |
| Talc | 12,5 | mg |
| Magnesium stearate | 2,5 | mg |
| | 250,0 | mg |

| Hard gelatin capsules | |
|---|---|
| Title compound I | 100 mg |
| Lactose | 197 mg |
| Magnesium stearate | 3 mg |
| | 300 mg |

| Syrup | |
|---|---|
| Title compound I | 0,4 g |
| Sucrose | 45 g |
| Flavoring agent | 0,2 g |
| Sweetening agent | 0,1 g |
| Water to | 100 mL |

| Aerosol | |
|---|---|
| Title compound I | 4 g |
| Flavoring agent | 0.2 g |
| Propylene glycol to | 100 mL |
| Suitable propellent to | 1 unit |

| Injectable preparation | |
|---|---|
| Title compound I | 100 mg |
| Benzylic alcohol | 0.05 mL |
| Propylene glycol | 1 mL |
| Water to | 5 mL |

The following examples illustrate, but do not limit, the preparation of the compounds of the present invention.

## PREPARATION 1

### (±)-1-Docosen-5-ol.

A flame dried flask was charged with magnesium turnings (1.22 g, 50 mmol), a crystal of $I_2$ and anhydrous ether (100 mL). The mixture was energically stirred and several drops of 1-bromo-4-butene were added. Once the brown color disapeared, a solution of 1-bromo-4-butene (4.8 mL, 48 mmol) in ether (30 mL) was added and the mixture stirred at room temperature for 30 min. Then, a solution of *n*-octadecanal (4.6 g, 30 mmol) in ether (40 mL) was added. After the addition, the mixture was heated to reflux 1 h. The reaction was quenched by addition of a solution of ammonium chloride (100 mL, 10 in water). The ethereal layer was separated, dried with anhydrous sodium sulfate and evaporated. Purification by flash chromatography (ethyl acetate:hexane 1:5) yielded the desired product as a white powder (5.20 g, 53%).

mp: 54-55 °C; IR (KBr) $\nu$: 3401, 2919, 2848, 1638, 1463, 1347, 911 $cm^{-1}$; $^1H$ NMR (80 MHz, $CDCl_3$) $\delta$ - (TMS): 6.1-5.6 (m, 1H, =CH), 5.2-4.9 (m, 2H, =$CH_2$), 3.65 (m 1H CHOH), 2.4-2.0 (m, 2H, =CH-$CH_2$), 1.7-0.8 (m, 37H).

*Analysis* calcd. for $C_{22}H_{44}O$ : C 81.41%; H 13.66%. Found: C 81.46%; H 14.05%.

## PREPARATION 2.

### (±)-1-Eicosen-5-ol

Following the procedure described in preparation 1, but using *n*-hexadecanal instead of *n*-octadecanal, the title compound of this preparation was obtained in a similar yield.

mp: 47-48 °C; IR (KBr) $\nu$: 3323, 2913, 2848, 1636, 1462, 1349, 913 $cm^{-1}$; $^1H$ NMR (80 MHZ, $CDCl_3$) $\delta$ - (TMS): 6.1-5.6 (m, 1H, =CH), 5.2-4.9 (m, 2H, =$CH_2$), 3.65 (m, 1H CHOH), 2.4-2.0 (m, 2H, =CH-$CH_2$), 1.7-0.8 (m, 33H).

*Analysis* calcd. for $C_{20}H_{40}O$ : C 81.01 %, H 13.59%. Found: C 80.82%; H 13.79%.

## PREPARATION 3

**(±)-6-Benzyloxy-1-hexen-5-ol**

Following the procedure described in preparation 1, but using benzyloxyethanal instead of *n*-octadecanal, the title compound of this preparation was obtained as an oil in a similar yield.
IR (KBr) ν: 3442, 3061, 3027, 2913, 2855, 1637, 1449, 1362, 1096, 910 $cm^{-1}$; $^1H$ NMR (80 MHz, $CDCl_3$) δ - (TMS): 7.33 (s, 5H, Ar), 6.1-5.6 (m, 1H, =CH), 5.2-4.9 (m, 2H, $=CH_2$), 4.55 (s, 2H, $Ar\underline{CH_2}$),3.8 (m, 1H, $\underline{CH}OH$), 3.6-3.2 (m, 2H, $CH_2O$), 2.4-2.0 (m,2H, = $CH\underline{CH_2}$), 1.7-1.4 (m, 2H, $=CHCH_2\underline{CH_2}$)
*Analysis* calcd. for $C_{13}H_{18}O_2$ : C 75.69% H 8.79%. Found: C 75.88% H 8.61%.

## PREPARATION 4

**(±)-*cis,trans*- 5-(heptadecyl)tetrahydrofuran-2-methanol.**

A flame dried flask was charged with 55% *m*-chloroperbenzoic acid (3.35 g, 10.6 mmol) and dichloromethane (25 mL). The aqueous layer was separated and the organic layer dried with anhydrous sodium sulfate and filtered. The organic solution was cooled to 0 °C and 1-docosen-5-ol (2.67 g, 8.22 mmol) obtained in the preparation 1, was added. The mixture was stirred at room temperature overnight Then, a solution of 10% sodium thiosulfate in water (2x 25 mL) was added and the mixture was vigorously stirred. The organic layer was washed with 10% solution of sodium hydroxide in water (3x 15 mL), dried over anhydrous sodium sulfate and evaporated, yielding a white powder. The product is pure enough to be used in the next step without further purification, however, an analytical sample can be obtained by flash chromatography (ethyl acetate:hexane 1:2). Yield : 1.54 g, 55%.
IR (KBr) ν: 3367, 2914, 2845, 1464, 1096, 1073, 1042 $cm^{-1}$; $^1H$ NMR (80 MHz, $CDCl_3$) δ (TMS): 3.9 (m, 2H, CHOCH), 3.7-3.4 (m, 2H, $CH_2OH$), 2.1-0.8 (m, 39H)
*Analysis calcd.* for $C_{22}H_{44}O_2$ : C 77.58%; H 13.02%. Found: C 77.89%; H 13.33%.

## PREPARATION 5

**(±)-*cis,trans*- 5-(pentadecyl)tetrahydrofuran-2-methanol**

Following the procedure described in preparation 4, but using 1-eicosen5-ol instead of 1-docosen-5-ol, the title compound of this preparation was obtained as a white solid in a similar yield.
m.p. 29-31 °C; IR (KBr) ν: 3389, 2916, 1462, 1100, 1044 $cm^{-1}$; $^1H$ NMR (80 MHz, $CDCl_3$) δ (TMS): 3.9 (m, 2H, CHOCH), 3.7-3.4 (m, 2H, $CH_2OH$), 2.1-0.8 (m, 39H).
*Analysis* calcd. for $C_{20}H_{40}O_2$ : C 76.86%; H 12.90%. Found: C 76.71%; H 12.91%.

## PREPARATION 6

**(±)-*cis, trans*- 5((benzyloxy)methyl)tetrahydrofuran-2-methanol**

Following the procedure described in preparation 4, but using 6-benzyloxy-1-hexen-5-ol instead of 1-docosen-5-ol, the title compound of this preparation was obtained as a colorless oil in a similar yield.
IR (film) ν: 3431, 3058, 3025, 2865, 1491, 1449, 1362, 1075, 738, 698 $cm^{-1}$; $^1H$ NMR (80 MHz, $CDCl_3$) δ - (TMS): 7.32 (s, 5H, Ar), 4.57 (s, 2H, $Ar\underline{CH_2}$), 4.15 (m, 2H, CHOCH), 3.9-3.4 (m, 4H, $BnO\underline{CH_2}$, $\underline{CH_2}OH$), 2.3-1.6 (m, 4H, $CH_2CH_2$)

*Analysis* calcd. for $C_{13}H_{18}O_3$ : C 70.24%; H 8.16%. Found: C 70.66%; H 8.17%.

## PREPARATION 7

**(±)-*cis,trans*-(((N-heptacecylcarbamoyl)oxy)methyl)-2-benzyloxy-tetrahydrofuran.**

In a flask, 5((benzyloxy)methyl)tetrahydrofuran-2-methanol (1.0 g, 4.5 mmol) obtained according to the preparation 6, was solved in dry pyridine (4 mL). To this solution, heptadecylisocyanate (2.4 g, 8.5 mmol) was added, and the mixture was heated at 70°C during 3 .5 h. Chloroform (50 mL) was added and the solution was washed first with a solution of hydrochloric acid (4.5 mL) in water (20 mL) ,then with a 10% solution of sodium bicarbonate (20 mL) and finally with saturated solution of sodium chloride (20 mL). The organic phase was dried over anhydrous sodium sulfate and concentrated. The crude was purified by flash chromatography yielding the desired product as a white solid (1.35 g, 60%)
mp: 61-62°C; IR (KBr) $\nu$: 3344, 3029, 2917, 2845, 1677, 1525, 1465, 1299, 1279, 1260, 1244, 1230, 1123 $cm^{-1}$; $^1H$ NMR (80 MHz, $CDCl_3$) $\delta$ (TMS): 7.32 (s, 5H, Ar), 4.7 (m, 1H, NH), 4.57 (s, 2H, $ArCH_2$), 4.4-4.0 (m, 4H, CHOCH, $C=COCH_2$), 3.49 (d, J=5.0 Hz, 2H, $BnOCH_2$), 3.15 ( broad q , J=6 Hz, 2H, $NCH_2$), 2.3-0.8 (m, 37H).
*Analysis* calcd. for $C_{31}H_{53}NO_4$ : C 73.91%; H 10.60%; N 2.78%. Found: C 73.50%, H 10.98%; N 2.55%.

## PREPARATION 8

**(±)-*cis,trans*-5-(((Npentadecylcarbamoyl)oxy)methyl)-2-benzyloxy-tetrahydrofuran**

Following the procedure described in preparation 7, but using pentadecylisocyanate instead of heptadecylisocyanate, the title compound of this preparation was obtained as a white solid in a similar yield.
mp: 54-56°C; IR (KBr) $\nu$: 3342, 3029, 2916, 2846, 1676, 1525, 1479, 1314, 1293, 1272, 1252, 1236, 1122 $cm^{-1}$; $^1H$ NMR (80 MHz, $CDCl_3$) $\delta$ (TMS): 7.31 (s, 5H, Ar), 4.8 (m, 1H, NH), 4.56 (s, 2H, $ArCH_2$), 4.4-3.9 (m, 4H, $O=COCH_2CHOCH$), 3.48 (d, J=5.2Hz, 2H, $BnOCH_2$), 3.35 (q, J=6Hz, 2H, $NCH_2$), 2.2-0.7 (m, 33H)
*Analysis* calcd. for $C_{29}H_{49}NO_4$ : C 73.22%; H 10.38%; N 2.94%. Found: C 73.17%; H 10.68%; N 2.71%.

## PREPARATION 9

**(±)*cis,trans*-5-[((N-octadecylcarbamoyl)ory)methyl]-2-benzyloxytetrahydrofuran**

Following the procedure described in preparation 7, but using octadecylisocyanate instead of heptadecylisocyanate, the title compound of this preparation was obtained as a white solid in a similar yield.
mp: 68-70°C; IR (KBr) $\nu$: 3343, 3029, 2918, 2845, 1677, 1525, 1470, 1299, 1273, 1255, 1236, 1132 $cm^{-1}$; $^1H$ NMR (80 MHz, $CDCl_3$) $\delta$ (TMS): 4.31 (s, 5H, Ar), 4.8 (m, 1H, NH), 4.57 (s, 2H, $ArCH_2$), 4.4-4.0 (m, 4H, $O=COCH_2CHOCH$), 3.50 (d, J=5Hz, 2H, $BnOCH_2$), 3.30 (broad q, J=6Hz, 2H, $NCH_2$), 2.2-0.7 (m, 39H)..
*Analysis* calcd. for $C_{33}H_{55}NO_4$ : C 74.23%; H 10.71%; N 2.70%. Found: C 74.44%; H 10.88%; N 2.81%.

## PREPARATION 10

**(±)-*cis, trans*- 5-[((N-heptadecylcarbamoyl)oxy)methyl]tetrahydrofuran-2-methanol**

A hydrogenator was charged with the compound obtained in the preparation 7 (1.26 g, 2.5 mmol), dichloromethane (20 mL), ethanol (2 mL) and 5% Pd/C (170 mg) The mixture was hydrogenated 18 h at 50

psi, and then, filtered and evaporated to afford the desired product as a white solid (880 mg, 85%) mp: 73-74 °C; IR (KBr) ν: 3341, 2916, 2846, 1677, 1525, 1479, 1279, 1259, 1244, 1230 $cm^{-1}$; $^1H$ NMR (80 MHz, $CDCl_3$) δ (TMS): 4.73 (m, 1H, NH), 4.3-3.9 (m, 4H, $O=COCH_2CHOCH$), 3.8-3.4 (m, 2H, $CH_2OH$), 3.16 (broad q, 2H, $CH_2N$), 2.2-0.8 (m,37H)
*Analysis* calcd. for $C_{24}H_{47}NO_4$ : C 69.69%; H 11.45%; N 3.38%. Found: C 70.10%; H 11.74%; N 3.02%.

**PREPARATION 11**

**(±)-*cis, trans*- 5-[((Npentadecylcarbamoyl)oxy)methyl]tetrahydrofuran-2-methanol**

**PREPARATION 12**

**(±)-*cis, trans*- 5-[((N-octadecylcarbamoyl)oxy)methyl]tetrahydrofuran-2-methanol**

Following the procedure described in preparation 10, but using the product obtained in preparation 8 as starting material, the title compound of this preparation was obtained in a similar yield.
mp: 77-79 °C; IR (KBr) ν: 3338, 2916, 2845, 1677, 1525, 1479, 1315, 1270, 1244, 1230, 1031 $cm^{-1}$.

PREPARATION 13

**(±)-*cis, trans*- 5-[((hexadecyl)oxy)methyl]-2-(benzyloxy)tetrahydrofuran**

To a cooled suspension (0 °C) of 55% sodium hydride (0.353 g, 8.09 mmol) in DMF (10 mL), was added a solution of the compound obtained in preparation 6 (1.05 g, 6.75 mmol) in DMF (5 mL) The mixture was stirred 5 min. and then, a solution of hexadecyl bromide (2.26 g, 7.42 mmol) in DMF (8 mL) was slowly added. The mixture was stirred 1h at 70 °C and 18 h at room temperature. The mixture was poured into cool water and diethylether was added. The ethereal layer was separated and the aqueous layer extracted with more ether (5x 20 mL). The combined organic layers were dried with anhydrous sodium sulfate and, after evaporation, 3.1g of an oil were obtained. Purification by flash chromatography (ethyl acetate:hexane 1:8) afforded the title compound of the preparation as a colorless oil (1.74 g, 58%).
IR (film) ν: 3026, 2919, 2849, 1463, 1362, 1093, 1027, 733, 696 $cm^{-1}$; $^1H$ NMR (80 MHz, $CDCl_3$) δ (TMS): 7.31 (s, 5H, ar), 4.56 (s, 2H, $ArCH_2$), 4.4-3.9 (m, 2H, CHOCH), 3.45 (t, 1 = 5.3Hz, 6H, $CH_2OCH_2$, $BnOCH_2$), 2.2-0.7 (m, 35H).

**PREPARATION 14**

**(±)-*cis,trans*- 5-[((octadecyl)oxy)methyl]-2-(benzyloxy)tetrahydrofuran**

Following the procedure described in preparation 13, but using octadecyl bromide instead of hexadecyl bromide the title compound of this preparation was obtained in a similar yield.
IR (KBr) ν: 3028, 2918, 2849, 1462, 1362, 1094, 1029, 733, 695 $cm^{-1}$; $^1H$ NMR (80 MHz, $CDCl_3$) δ (TMS): 7.32 (s, 5H, Ar), 4.56 (s, 2H, $ArCH_2$), 4.4-3.9 (m, 2H, CHOCH), 3.44 (t, J = 5.3 Hz, 6H, $CH_2OCH_2$, $BnOCH_2$), 2.2-0.7 (m, 39H).

**PREPARATION 15**

**(±)-cis,trans- 5-[((hexadecyl)oxy)methyl]tetrahydrofuran-2-methanol**

Following the procedure described in preparation 10, the compound obtained in praparation 13 was hydrogenated to afford the title compound as a colorless oil in a similar yield.
IR (film) $\nu$: 3421, 2917, 2847, 1636, 1463, 1375, 1075, 947, 883, 721 $cm^{-1}$; $^1H$ NMR (80 MHz, $CDCl_3$) $\delta$ (15): 4.3-3.9 (m, 2, CHOCH), 3.9-3.3 (m, 6H, $CH_2OCH_2$), 2.2-0.7 (m, 35H).

## PREPARATION 16

**(±)-*cis,trans*- 5-[((octadecyl)oxy)methyl]tetrahydrofuran-2-methanol**

Following the procedure described in preparation 10, the compound obtained in praparation 14 was hydrogenated to afford the title compound as a colorless oil in a similar yield.
IR (film) $\nu$: 3420, 2918, 2847, 1636, 1465, 1377, 1074, 947, 884, 722 $cm^{-1}$; $^1H$ NMR (80 MHz, $CDCl_3$) $\delta$ - (TMS): 4.3-3.9 (m, 2H, CHOCH), 3.9-3.4 (m, 6H, $CH_2OCH_2$, $HOCH_2$), 2.2-0.7 (m, 39H).

## EXAMPLE 1

**(±)-*cis,trans*- 2-[N-[((5-heptadecyl-2-tetrahydrofuranyl)methoxy)carbonyl] aminomethyl] pyridine**

A flame dried flask was charged with the compound obtained in preparation 4 (1.50 g, 4.25 mmol), dry dichloromethane (20 mL) and dry pyridine (0.68 mL, 8.5 mmol). The solution was cooled at 0° C and phenyl chlorocarbonate (0.60 mL, 4.68 mmol) was slowly added. The mixture was stirred 1h at 25° C., then, dichloromethane was added (20 mL) and the solution was washed with 1N aqueous HCl solution. The solution was dried over anhydrous sodium sulfate, filtered and concentrated to afford the corresponding mixed carbonate (2.05 g, 104 This substance (1.90 g, 4.12 mmol), was solved, in dry chloroform (5 mL) and, then, 2-picolylamine (0.5 mL, 4.94 mmol) was added. The reddish solution was stirred at reflux for 18 h. Then, dichloromethane was added (20 mL) and the solution washed with 1N NaOH solution (3x15 mL), followed by brine. The organic layer was dried over anhydrous sodium sulfate, filtered and concentrated to afford the desired product as a brown semisolid (2.02 g, 103%). In spite of its color, TLC analysis shows that the substance is pure enough to be used in the next step
IR (KBr) $\nu$: 3311, 3054, 2914, 2843, 1673, 1588, 1537, 1466, 1303, 1289, 1272, 1260, 1098, 1053 $cm^{-1}$; $^1H$ NMR (80 MHz, $CDCl_3$) $\delta$ (TMS): 8.55 (dq, 1H, pyr), 7.65 (dt, $J_d$ = 1.7 Hz, 1H = 7.3 Hz, 1H, pyr), 7.4-7.1 (m, 2H, pyr), 5.8 (m, 1H, NH), 4.49 (d, 1 = 5.5 Hz, 2H, $CH_2N$), 4.3-3.8 (m, 4H, $OCH_2CHOCH$), 2.2-0.75 (m,39H).

## EXAMPLE 2

**(±)-*cis,trans*- 2-[N-acetyl-N-[((5-heptadecyl-2-tetrahydrofuranyl)methoxy) carbonyl] aminomethyl] pyridine**

In a dry flask, the compound obtained in example 1 (900 mg, 1.89 mmol), was solved in dry dicloromethane (10 mL). The solution was cooled at 0° C and acetyl chloride (0.17 mL, 2.46 mmol) was slowly added. The reaction mixture was stirred 1.5h at 0° C and 48 h at room temperature. Then, triethylamine was added, the mixture was diluted with dichloromethane and washed with water (2x 20 mL). The organic layer was dried over anhydrous sodium sulfate, filtered and concentrated to afford a brown solid (1.08 g) which after flash chromatography purification (ethyl acetate: hexane 1:1) yielded the desired

compound as a white solid (641 mg, 70% three steps)
mp: 45-46° C; IR (KBr) ν: 2915, 2848, 1737, 1693, 1589, 1373, 1338, 1283, 1226, 1094 $cm^{-1}$; $^1H$ NMR (80 MHz, $CDCl_3$) δ (TMS): 8.49 (dq, 1H, pyr), 7.65 (dt, $J_d$ = 1.7 Hz, $J_t$ = 7.3 Hz, 1H, pyr), 7.2-7.0 (m, 2H, pyr), 5.10 (s, 2, $CH_2N$), 4.2-3.6 (m, 4H, $OCH_2CHOCH$), 2.63 (s, 3H, $CH_3$), 2.2-0.75 (m, 39H).
*Analysis* calcd. for $C_{31}H_{52}N_2O_4$ : C 72.05%; H 10.14%; N 5.42%. Found: C 71.71%; H 9.91%; N 5.02%.

## EXAMPLE 3

**(±)-*cis,trans*- 2-[N-acetyl-N-[((5-heptadecyl-2-tetrahydrofuranyl)methoxy) carbonyl] aminomethyl]-1-ethylpiridinium iodide**

A solution of the compound obtained in example 2 (401 mg, 0.77 mmol) in acetonitrile (1 mL) and ethyl iodide (1 mL) was heated at 70° C three days. The solution was concentrated *in vacuò*, the residue was solved in dichoromethane and precipitated with ether. The solid was filtered and dried to yield the desired compound as a yellowish powder (450 mg, 87%)
mp: 47-50° C; IR (KBr) ν: 3340 ($H_2O$), 2914, 2848, 1732, 1677, 1625, 1579, 1509, 1464, 1367, 1210, 1162, 1084, 985, 772 $cm^{-1}$; $^1H$ NMR (80 MHz, $CDCl_3$) δ (TMS): 9.73 (d, 1 = 6 Hz, 1H, pyr), 8.49 (dt, $J_d$ = 1.6 Hz, $J_t$ = 7.5 Hz, 1H, pyr), 8.08 (t, J = 7.5 Hz, 1H, pyr), 7.81 (d, 1 = 8.5 Hz, 1H, pyr), 5.43 (s, 2H, $CH_2N$), 5.09 (q, J = 7.5 Hz, 2H, $NCH_2CH_3$), 4.4-3.7 (m, 4H, $OCH_2CHOCH$), 2.67 (s, 3H, $CH_3$), 1.74 (t, 1 = 7.5 Hz, 3H, $NCH_2CH_3$), 2.2-0.7 (m, 39H).
*Analysis* calcd. for $C_{33}H_{57}IN_2O_4$ .1.4$H_2O$: C 56.78%; H 8.63%; N 4.01%. Found: C 56.61%; H 8.51%; N 3.86%.

## EXAMPLE 4

**(±)-*cis,trans*- 2-[N-acetyl-N-[((5-heptadecyl-2-tetrahydrofuranyl)methoxy) carbonyl] aminomethyl]-1-ethylpiridinium chloride**

A solution of the compound obtained in example 3 (136 mg, 0.20 mmol) in 70% aqueous methanol (150 mL) was eluted through an ion-exchange resin column IRA-410 (chloride formation). The filtrate was concentrated to dryness and recrystallized in acetone-ether to yield the title compound as a white powder (124 mg, 95%).
mp: 55-65° C; IR (KBr) ν: 3425 ($H_2O$), 2914, 2846, 1730, 1679, 1580, 1463, 1367, 1214, 1165, 1086, 987, 773, $cm^{-1}$; $^1H$ NMR (80 MHz, $CDCl_3$) δ (TMS): 10.32 (broad d, 1H, pyr), 8.35 (broad d, 1H, pyr), 8.07 (m, 1H, pyr), 7.7 (broad d, 1H, pyr) 5.40 (s, 2H, $CH_2N$), 5.24 (q, J = 7.0 Hz, 2H, $NCH_2CH_3$), 4.4-3.7 (m, 4H, $OCH_2CHOCH$),2.66 (s, 3H, $CH_3$), 2.5-0.8 (m, 39H).
*Analysis* calcd. for $C_{33}H_{57}ClN_2O_4$ .1 $H_2O$: C 66.14% H 9.92%; N 4.67%. Found: C 65.97%; H 9.91%; N 4.48%.

## EXAMPLE 5

**(±)-*cis,trans*-2-[N-acetyl-N-[((5-heptadecyl-2-tetrahydrofuranyl)methoxy) carbonyl] aminomethyl]-1-methylpiridinium iodide**

Follwing the procedure described in example 3, but using methyl iodide instead of ethyl iodide, and heating to 70° C during 18 h instead of 3 days, the title compound of this example was obtained as a yellowish solid in a similar yield.
mp: 87-92° C; IR (KBr) ν: 3425 ($H_2O$), 2916, 2847, 1707, 1625, 1578, 1508, 1463, 1412, 1380, 1365, 1342, 1213, 979 $cm^{-1}$; $^1H$ NMR (80 MHz, $CDCl_3$) δ (TMS): 9.50 (broad d, 1H, pyr), 8.45 (broad t, 1H, pyr) 8.1-7.8

(m, 2H, pyr). 5.41 (s, 2H, $CH_2N$), 4.72 (s, 3H, $NCH_3$), 4.4-3.8 (m, 4H, $OCH_2CHOCH$), 2.64 (s, 3H, $CH_3$), 2.2-0.7 (m, 39H).
*Analysis* calcd. for $C_{32}H_{55}IN_2O_4$ . 0.5 $H_2O$: C 57.56%; H 8.45%; N 4.19%. Found: C 57.51%; H 8.60%, N 4.12%.

## EXAMPLE 16

**(±)-*cis,trans*-2-[N-ethoxycarbonyl-N-[((5-heptadecyl-2-tetrahydrofuranyl) methoxy) carbonyl] aminomethyl] pyridine**

Following the procedure described in example 1e, but using ethyl chlorocarbonate instead of acetyl chloride, the title compound of this example was obtained as a pasty solid in a similar yield.
IR (KBr) $\nu$: 2920, 2850, 1793, 1754, 1722, 1592, 1462, 1434, 1371, 1338, 1296, 1204 $cm^{-1}$; $^1H$ NMR (80 MHz, $CDCl_3$) $\delta$ (TMS): 8.52 ($d_q$,$J_q$ = 1.1 Hz, $J_d$ = 4.8 Hz, 1H, pyr) 7.55 (dt, $J_d$ = 1.7 Hz, $J_t$ = 7.3 Hz, 1H, pyr), 7.3-7.0 (m, 2H, pyr), 5.04 (s, 2H, $CH_2N$), 4.26 (q, J = 7.1 Hz, 2H, $OCH_2CH_3$), 4.3-3.6 (m, 4H, $OCH_2CHOCH$), 2.2-0.7 (m,39H).
*Analysis* calcd. for $C_{32}H_{54}N_2O_5$ : C 70.29%; H 9.95%; N 5.12%. Found: C 70.08%; H 9.97%; N 4.81%.

## EXAMPLE 7

**(±)-*cis,trans*-2-[N-ethoxycarbonyl-N-[((5-heptadecyl-2-tetrahydrofuranyl) methoxy) carbonyl] aminomethyl]-1-ethylpyridinium iodide**

Following the procedure described in example 3, but using the substance obtained in example 6 instead of the one obtained in example 2, the title compound of this example was obtained as a yellow solid in a similar yield.
mp: 45-46 °C; IR (KBr) $\nu$: 3441 ($H_2O$), 2913, 2846, 1789, 1758, 1693, 1625, 1579, 1509, 1463, 1422, 1370, 1338, 1297, 1212, 1165, 1095, $cm^{-1}$ $^1H$ NMR (80 MHz, $CDCl_3$) $\delta$ (TMS): 9.87 (broad d, J = 6.5 Hz, 1H, pyr), 8.45 (broad t, J = 6.5 Hz, 1H, pyr), 8.11 (broad t, J = 6.5 Hz, 1H, pyr), 7.83 (broad d, J = 7.7 Hz, 1H, pyr), 5.40 (s, 2H, $CH_2N$), 5.10 (q, J = 7.3 2H, Hz, 2H, $NCH_2CH_3$), 4.34 (q, J = 7.2 Hz, 2H, $OCH_2$), 4.3-3.8 (m, 4H, $OCH_2CHOCH$), 2.2-0.8 (m, 45H).
*Analysis* calcd. for $C_{34}H_{59}IN_2O_5$ . $2H_2O$: C 55.27%; H 8.59%; N 3.79%. Found: C 55.25%; H 8.21%; N 3.63%.

## EXAMPLE 8

**(±)-*cis,trans*-2-[N-methyl-N-[((5-heptadecyl-2-tetrahydrofuranyl)methoxy) carbonyl] aminomethyl]-pyridine**

To a cooled solution (-78 °C) of the compound obtained in example 1 (0.5 g, 1.05 mmol) in dry tetrahydrofuran (5 mL) was added a solution of n-butyl lithium 1.6M in hexane (0.72 mL, 1.16 mmol) dropwise. The mixture was allowed to warm to 0 °C and stirred at this temperature 15 min. Then, the solution was cooled to -65 °C and methyl iodide (0.45 g, 3.16 mmol) was added. The mixture was then stored at 5 °C 3 days and quenched by addition of a saturated solution of amonium chloride in water. The mixture was concentrated *in vacuo* and treated with water and dichloromethane. The organic layer was separated, dried over anhydrous sodium sulfate, filtered and concentrated to afford 570 mg of a reddish viscous liquid. Purification by flash chromatography yielded pure product (100 mg, 20%) as a colorless oil.
IR (film) $\nu$: 2919, 2849, 1702 1589, 1567, 1463, 1400, 1304, 1230, 1212, 1142, 733 $cm^{-1}$; $^1H$ NMR (80 MHz, $CDCl_3$) $\delta$ (TMS): 8.54 (dq, $J_q$ = 1.1 Hz, $J_d$ = 4.7 Hz, 1H, pyr), 7.63 (dt, $J_d$ = 2 Hz, $J_t$ = 7.7 Hz, 1H, pyr), 7.2-7.1

(m, 2H, pyr), 4.60 (s, 2H, $CH_2N$), 4.4-3.5 (m, 4H, $OCH_2CHOCH$), 2.98 (s, 3H, $NCH_3$), 2.2-0.7 (m, 39H).

## EXAMPLE 9

### (±)-*cis,trans*-2-[N-[((5-pentadecyl-2-tetrahydrofuranyl)methoxy)carbonyl] aminomethyl] pyridine

Following the procedure described in example 1, but using 5-(pentadecyl)tetrahydrofuran-2-ol (obtained according preparation 5) instead of 5-(heptadecyl)tetrahydrofuran-2-ol, the title compound of this example was obtained in a similar yield .

IR (KBr) $\nu$: 3309, 3048, 2921, 2849, 1714, 1589, 1567, 1530, 1463, 1433, 1259, 1205, 1147, 1094, 1047, 996, 753 $cm^{-1}$; $^1H$ NMR (80 MHz, $CDCl_3$) $\delta$ (TMS): 8.55 (dq, 1H, pyr), 7.65 (dt, $J_d$ = 1.7 Hz, $J_t$ = 7.3 Hz, 1H, pyr), 7.4-7.1 (m, 2H, pyr), 5.8 (m, 1H, NH), 4.49 (d, J = 5.5 Hz, 2H, $CH_2N$), 4.3-3.8 (m, 4H, $OCH_2CHOCH$), 2.2-0.75 (m, 35H).

## EXAMPLE 10

### (±)-*cis,trans*-2-[N-acetyl-N-[((5-pentadecyl-2-tetrahydrofuranyl)methoxy) carbonyl] aminomethyl] pyridine

Following the procedure described in example 2, but using the product obtained in example 9 instead of the one obtained in example 1, the title compound of this example was obtained as a white solid in a similar yield.

mp: 36-38 °C; (KBr) $\nu$: 2913, 2846, 1736, 1693, 1588, 1463, 1429, 1368, 1338, 1225, 1096, 1079, 1042, 976 $cm^{-1}$; $^1H$ NMR (80 MHz, $CDCl_3$) $\delta$ (TMS): 8.49 (dq, 1H, pyr), 7.65 (dt, $J_d$ = 1.7 Hz, $J_t$ = 7.3 Hz, 1H, pyr), 7.2-7.0 (m, 2H, pyr), 5.10 (s, 2H, $CH_2N$), 4.2-3.6 (m,4H, $OCH_2CHOCH$), 2.63 (s, 3H, $CH_3$), 2.2-0.75 (m, 35H).

*Analysis* calcd. for $C_{29}H_{48}N_2O_4$ : C 71.27%; H 9.90%; N 5.73%. Found: C 71.08%; H 9.9%; N 5.33%.

## EXAMPLE 11

### (±)-*cis,trans*-2-[N-acetyl-N-[((5-pentadecyl-2-tetrahydrofuranyl)methoxy) carbonyl] aminomethyl]-1-ethylpyridinium iodide

Following the procedure described in example 3, but using the product obtained in example 10 instead of the one obtained in example 2, the title compound of this example was obtained as a yellow powder in a similar yield.

mp: 82-110 °C; IR (KBr) $\nu$-: 3459 ($H_2O$), 2914, 2847, 1742, 1687, 1625, 1580, 1509, 1481, 1463, 1447, 1411, 1362, 1229, 1203, 1082, 989 $cm^{-1}$; $^1H$ NMR (80 MHz, $CDCl_3$) $\delta$ (TMS): 9.74 (broad d, J = 6 Hz, 1H, pyr), 8.47 (broad t, J = 7.7 Hz, 1H, pyr), 8.07 (broad t,J = 6.3 Hz, 1H, pyr), 7.8 (broad d, J = 7.7 Hz, 1H, pyr), 5.42 (s, 2H, $CH_2N$), 4.8 (q, J = 7.5 Hz, 2H, $NCH_2CH_3$), 4.4-3.7 (m, 4H, $OCH_2CHOCH$), 2.66 (s, 3H, $CH_3$), 1.74 (t, J = 7.5 Hz, 3H, $NCH_2CH_3$), 2.2-0.7 (m, 35H).

*Analysis calcd.* for $C_{31}H_{53}IN_2O_4$ : C 57.75%; H 8.28%; N 4.34%. Found: C 57.89%; H 8.31%; N 4.14%.

## EXAMPLE 12

### (±)-*cis,trans*-3-[6-[(5-pentadecyl-2-tetrahydrofuranyl)methoxy]-6-oxohexyl] tiazolium 4-methylbenzenesulfonate

a) (±)-*cis,trans*- 6-[5-pentadecyl-2-tetrahydrofuranyl)methoxy]-6-oxohexyl] 4-methylbenzenesulfonate

To a cooled solution (0 °C) of the compound obtained in preparation 5 (300 mg, 0.96 mmol) in dichoromethane (8 mL) and pyridine (0.23 mL, 2.88 mmol) was added 4-N,N-dimethylaminopyridine (8mg) followed by 6-tosyloxyhexanoyl chloride (0.8 g, 1.37 mmol) in dichloromethane (3 mL). The reaction mixture was stirred at room temperature 18 h. Then, was poured over slightly acidified water (HCl, pH = 2) and extracted with dichloromethane (3 mL). The organic layer was washed with 10% solution of sodium bicarbonate in water and brine, dried over anhydrous sodium sulfate, filtered and concentrated. The crude reaction was purified by flash chromatography (ethyl acetate:hexane 1:3) to yield the desired compound as a colorless oil.
IR (KBr) $\nu$: 2923, 2851, 1730, 1595, 1491, 1461, 1361, 1187, 1176, 1097,952, 814, 662, 554 $cm^{-1}$ ; $^1$H NMR (80 MHz, $CDCl_3$) $\delta$ (TMS): 7.78 (d, J = 8.3 Hz, 2H, Tos), 7.34 (d, J = 8.3 Hz, 2H, Tos), 4.3-3.8 (m, 6H, $TsOCH_2$, $OCH_2CHOCH$,), 2.44 (s, 3H, $TosCH_3$), 2.29 (t, J = 7 Hz, 2H, O = $CCH_2$), 2.0-0.7 (m, 41H).

b) Preparation of the title compound.

A solution of the compound obtained in example 12a (450 mg, 0.77 mmol) in tiazol (1.5 mL) was heated at 80 °C 8 h. under argon atmosphere. The mixture was concentrated to dryness and the remaining solid was dissolved in the minimum amount of dichloromethane and was precipitated with ether. The solid obtained was filtered and vacuum-dried to yield the title compound of the example as a cream-colored solid (461 mg, 89%)
mp: 83.4-85.7 °C; IR (KBr) $\nu$: 3137, 2913, 2845, 1734, 1559, 1463, 1380, 1296, 1208, 1193, 1142, 1121, 1099, 1035, 1011, 817, 684, 560 $cm^{-1}$; $^1$H NMR (80 MHz $CDCl_3$) $\delta$ (TMS): 10.8 (s, 1H, NCHS), 8.3 (m, 2H, NCHCHS), 7.76 (d, J = 8.1 Hz, 2H, Tos), 7.15 (d, J = 8.1 Hz, 2H, Tos), 4.67 (t, J = 7.2 Hz, 2H, $NCH_2$), 4.4-3.7 (m, 4H, $OCH_2CHOCH$), 2.34 (s, 3H, $ArCH_3$), 2.4-0.8 (m, 43H).
*Analysis* calcd. for $C_{36}H_{59}NO_6S_2$: C 64.92%; H 8.93%; N 2.1%. Found: C 65.03%; H 9.11%; N 1.99%.

## EXAMPLE 13

**(±)-*cis,trans*-3-[6-[(5-pentadecyl-2-tetrahydrofuranyl)methoxy]hexyl]tiazolium 4-methylbenzenesulfonate**

a)(±)-*cis,trans*-5-pentadecyl-2-(((6(benzyloxy)hexyl)oxy)methyl)tetrahydrofuran

55% of sodium hydride in oil (0.24 g, 5.57 mmol) was suspended in dry DMF (5 mL). The mixture was cooled to 0 °C and a solution of the substance obtained in preparation 5 (1.34 g, 4.28 mmol) in DMF (5 mL) was added causing the corresponding gas evolution (112). Then, 6-benzyloxyhexyl tosylate (2.02 g, 5.57 mmol) was added and the mixture was heated until the reaction started. The reaction was then stirred at room temperature 24 h, poured into cold water and extracted with ether (6x 25 mL). The combined ethereal phases was washed with brine, dried over anhydrous sodium sulfate and concentrated to yield a semi-solid residue which was purified by flash chromatography (ethyl acetate:hexane 1:7). The desired product was obtained as a colorless thick liquid (1.44 g, 67%).
IR (film) $\nu$: 3058, 3025, 2922, 2850, 1491, 1450, 1359, 1304, 1238, 1200, 1100, 902, 733, 696 $cm^{-1}$; $^1$H NMR (80 MHz, $CDCl_3$) $\delta$ (TMS): 7.31 (s, 5H, Ar), 4.49 (s, 2H, $ArCH_2$), 4.3-3.7 (m, 2H, CHOCH), 3.7-3.3 (m, 6H, $CH_2OCH_2$, $BnOCH_2$), 2.1-0.7 (m, 43H).

b) (±)-*cis, trans*- 6-(5-pentadecyl-2-tetrahydrofuranyl)methoxy)-1-hexanol

A suspension of the product obtained in example 13a (1.34 g, 2.66 mmol) and 5% paladium over carbon (0.2 g) in dichloromethane (30 mL) and ethanol (4 mL) was hydrogenated at 40 psi during 18 h. The mixture was filtered and concentrated to afford the desired product as a colorless thick liquid (905 mg, 86%).

IR (film) ν: 3416, 2920, 2850, 1461, 1375, 1120, 885, 721 $cm^{-1}$; $^1H$ NMR (80 MHz, $CDCl_3$) δ (TMS): 4.3-3.8 (m, 2H, CHOCH), 3.8-3.3 (m, 7H, $CH_2OCH_2$, $CH_2OCH$), 2.2-0.7 (m, 43H).

c) (±)-*cis,trans*- 6((2-pentadecyl-2-tetrahydrofuranyl)methoxy)hexyl 4-methylbenzenesulfonate

To a precooled (0 °C) solution of the compound obtained in example 13b (150 mg, 0.36 mmol) in dichloromethane (2 mL) and pyridine (0.5 mL, 6.35 mmol) was added p-toluensulfonyl chloride (90 mg, 0.47 mmol). The colorless mixture was stirred at room temperature 18 h, and then, poured over cold water. Dichloromethane was added, the organic layer was separated and washed succesively with a 1N HCl solution (20 mL), 10% sodium bicarbonate (20 mL) and brine (20 mL). The organic phase was dried over anhydrous sodium sulfate, filtered and concentrated *in vacuo* to afford a white semi-solid which is purified by flash chromatography (ethyl acetate:hexane 1:3) yielding the desired compound as a colorless thick oil (162 mg, 77%)
IR (KBr) ν: 2919, 2849, 1594, 1461, 1361, 1187, 1176, 1096, 964, 926, 814, 662, 573, 554 $cm^{-1}$; $^1H$ NMR (80 MHz, $CDCL_3$) δ (TMS): 7.78 (d, J=8.4 Hz, 2H, Tos), 7.65 (d, J=8.4 Hz, 2H, Tos), 4.2-3.9 (m,4H, $TsOCH_2$, CHOCH,),3.6-3.3 (m,4H, $CH_2OCH_2$) 2.45 (s, 3H, $TosCH_3$), 2.0-0.7 (m,43H).

d) Preparation of the title compound.

Following the procedure described in example 12b, but using the product obtained in example 13c instead of the one obtained in example 12a, the title compound of this example was obtained in a similar yield .
np: 77-80 °C; IR (KBr) ν: 3450 ($H_2O$), 3136, 2914, 2846, 1559, 1463, 1210, 1194, 1121, 1035, 1010, 818, 684, 561 $cm^{-1}$; $^1H$ NMR (80 MHz, $CDCl_3$) δ (TMS): 10.75 (m, 1H, NCHS), 8.2 (m, 2H, NCHCHS), 7.76 (d, J=81 Hz, 2H, Tos), 7.15 (d, J=81 Hz, 2H, Tos), 4.65 (t, J=7.2 Hz, 2H, $NCH_2$), 3.9 (m, 2H, CHOCH), 3.4 (m,4H, $CH_2OCH_2$), 2.34 (s, 3H, $ArCH_3$), 2.4-0.8 (m, 43H).
*Analysis* calcd. for $C_{36}H_{6}\cdot NO_5S_2$. 3/2 $H_2O$: C 63.68%; H 9.50%; N 2.06%. Found: C 63.32%; H 9.24%; N 2.31%.

## EXAMPLE 14

**(±)-cis,trans-2-[N-[((5-(((N-heptadecylcarbamoyl)oxy)methyl)-2-tetrahydrofuranyl) methoxy) carbonyl] aminomethyl] pyridine**

Following the procedure described in example 1, but using the product obtained in preparation 10 instead of the one obtained in preparation 4, the title compound of this example was obtained in a similar yield .
IR (KBr) ν: 3325, 2916, 2846, 1677, 1588, 1566, 1529, 1465, 1277, 1259, 1245, 1230, 1143, 1052, 925 $cm^{-1}$; $^1H$ NMR (80 MHz, $CDCl_3$) δ (TMS): 8.51 (broad d, J=4 Hz, 1H pyr), 7.66 (dt, $J_d$=1.8 Hz, $J_t$=7.6 Hz, 1H, pyr), 7.3-7.1 (m, 2H, pyr), 5.81 (m, 1H, NH), 4.7 (m, 1H, NH), 4.49, (d, J=7.1 Hz), 2H, $NCH_2Ar$), 4.3-3.9 (m, 6H), 3.14 (q, J=5.7 Hz, 2H, $NCH_2$), 2.2-0.7 (m, 37H).

## EXAMPLE 15

**(±)*cis,trans*-2-[N-acetyl-N-[((5(((N-heptadecylcarbamoyl)oxy)methyl)-2-tetrahydrofuranyl) methoxy)-carbonyl] aminomethyl] pyridine**

Following the procedure described in example 2, but using the product obtained in example 14 instead of the one obtained in example 1, the title compound of this example was obtained in a similar yield .
mp: 55-56 °C; IR (KBr) ν: 3364, 2913, 2845, 1737, 1682, 1594, 1525, 1463, 1434, 1397, 1373, 1345, 1289,

1259, 1214, 1155, 1099, 1074, 903 cm⁻¹; ¹H NMR (80 MHz, $CDCl_3$) δ (TMS): 8.49 (broad d, J=4.2 Hz, 1H, pyr), 7.64 (broad t, J=7.1 Hz, 1H, pyr), 7.3-7.0 (m, 2H, pyr), 5.4 (m, 1H, NH), 5.11 (s, 2H, $ArCH_2N$), 4.4-3.9 (m, 6H, $OCH_2CHOCHCH_2O$), 3.16 (q, J=6 Hz, 2H, $NCH_2$), 2.63 (s, 3H, $CH_3$), 2.2-0.7 (m, 37H).
*Analysis* calcd. for $C_{33}H_{55}N_3O_6$ : C 67.20%; H 9.40%; N 7.12%. Found: C 66.89%; H 9.62%; N 6.75%.

## EXAMPLE 16

**(±)-*cis,trans*-2-[N-acetyl-N-[((5-(((N-heptadecylcarbamoyl)oxy)methyl)-2-tetrahydrofuranyl)methoxy)-carbonyl] aminomethyl] -1-ethylpiridinium iodide**

Following the procedure described in example 3, but using the product obtained in example 15 instead of the one obtained in example 2, the title compound of this example was obtained as a yellow solid in a similar yield.
mp: 46-63 °C; IR (KBr) ν: 3335, 2913, 2846, 1750, 1692, 1625, 1579, 1524, 1367, 1223, 1160, 1085, 985, 772, cm⁻¹; ¹H NMR (80 MHz, $CDCl_3$) δ (TMS): 9.67 (broad t, J=4.7 Hz, 1H, pyr), 8.49 (dt, $J_d$=1.4 $H_t$=8.0 Hz, 1H, pyr), 8.05 (broad t, J=7 Hz, pyr), 7.81 (broad d, J=8 Hz), 5.43 (s, 2H, $ArCH_2N$), 5.09 (q, J=7.4 Hz, 2H $NCH_2CH_3$) 4.4-3.9 (m, 6H, $OCH_2CHOCHCH_2O$), 3.07 (q, J=6.6Hz, 2H, $NCH_2$), 2.67 (s, 3H, $CH_3$), 1.74 (t, J=7.4 Hz, 3H,. $NCH_2CH_3$), 2.3-0.7 (m, 37H).
*Analysis* calcd. for $C_{35}H_{60}IN_3O_6$ : C 56.37%, H 8.11%; N 5.63%. Found: C 56.64%; H 8.35%; N 5.61%.

## EXAMPLE 17

**(±)-*cis,trans*-2-[N-(((N-pentadecylcarbamoyl)oxy)methyl)-2-tetrahydrofuranyl) methoxy)carbonyl] aminomethyl]pyridine**

Following the procedure described in example 1, but using the product obtained in preparation 11 instead of the one obtained in preparation 4, the title compound of this example was obtained in a similar yield. Purification by flash chromatography afforded an analitical sample of this compound.
mp: 77-84 °C; IR (KBr) ν: 3328, 3053, 2916, 2846, 1676, 1588, 1529, 1464, 1442, 1253, 1142, 1055, 925 cm⁻¹. ¹H NMR (80 MHz, $CDCl_3$) δ (TMS): 8.53 (broad d, J=5.0 Hz, 1H, pyr), 7.66 (dt, $J_d$=1.6Hz, $J_t$=7.8Hz, 1H, pyr), 7.4-7.1 (m, 2H, pyr), 5.8 (m, 1H, NH), 4.8 (m, 1H, NH), 4.49 (d, J=5.4Hz, 2H, pyr-$CH_2$), 4.3-3.9 (m, 6H, $OCH_2CHOCHCH_2O$), 3.14 (q, J=6.3Hz, 2H, $NCH_2$), 2.2-0.7 (m, 33H).
*Analysis* calcd. for $C_{29}H_{49}N_3O_5$ : C 67.02%; H 9.50%; N 8.08%. Found: C 66.99%; H 9.58%; N 8.05%.

## EXAMPLE 18

**(±)-*cis,trans*-2-[N-acetyl-N-[((5-(((N-pentadecylcarbamoyl)oxy)methyl)-2-tetrahydrofuranyl) methoxy)carbonyl] aminomethyl]pyridine**

Following the procedure described in example 2, but using the product obtained in example 17 instead of the one obtained in example 1, the title compound of this example was obtained in a similar yield.
mp: 51-53 °C; IR (KBr) ν: 3363, 2913, 2845, 1737,1684, 1594, 1525, 1373, 1345, 1214, 1155, 1074, cm⁻¹; ¹H NMR (80 MHz, $CDCl_3$) δ (TMS): 8.52 (m, 1H, pyr), 7.63 (dt, $J_t$=7.0Hz, 1H, pyr), 7.3-7.0 (m, 2H, pyr), 5.3 (m, 1H, NH), 5.10 (s, 2H, pyr-$CH_2$), 4.3-3.8 (m, 6H, $OCH_2CHOCHCH_2O$), 3.15 (q, J=6.1Hz, 2H, $NCH_2$), 2.64 (s, 3H, $CH_3$), 2.2-0.7 (m, 33H).
*Analysis calcd.* for $C_{31}H_{49}N_3O_6$ : C 66.52%; H 8.82%; N 7.51%. Found: C 66.33%, H 9.21%; N 7.34%.

## EXAMPLE 19

**(±)-*cis,trans*-2-[N-acetyl-N-[((5-(((N-pentadecylcarbamoyl)oxy)methyl)-2-tetrahydrofuranyl)methoxy)-carbonyl] aminomethyl] -1-ethylpiridinium iodide**

Following the procedure described in example 3, but using the product obtained in example 18 instead of the one obtained in example 2, the title compound of this example was obtained as a pasty yellow solid in a similar yield .
IR (KBr) ν: 3321, 2918, 2848, 1745, 1696, 1624, 1578, 1518, 1518, 1446, 1366, 1212, 1161, 1085, $cm^{-1}$ $^1H$ NMR (80 MHz, $CDCl_3$) δ (TMS): 9.62 (m, 1H pyr), 8.51 (t, J=7.5 Hz, 1H, pyr), 8.05 (t, J=6.1Hz, 1H, pyr), 7.92 (d, J=8 Hz, 1H, pyr), 5.46 (s, 2H, pyr-$CH_2$), 5.4 (m, 1H, NH), 5.06 (q, J=7.3Hz, 2H, $NCH_2$), 2.67 (s, 3H, $CH_3$), 1.73 (t, J=7.3 Hz, 3H, $NCH_2CH_3$), 2.2-0.7 (m, 33H).
*Analysis* calcd. for $C_{33}H_{55}N_3O_6I$ . 1 2 $H_2O$: C 54.53%; H 7.91%; N 5.78%. Found: C 54.59%; H 7.78%; N 5.85%.

**EXAMPLE 20**

**(±)-*cis,trans*-2-[N-[((6-((5-pentadecyl-2-tetrahydrofuranyl)methoxy)hexyl) oxy)carbonyl] aminomethyl]pyridine**

Following the procedure described in example 1, but using the product obtained in example 13b instead of the one obtained in preparation 4, the title compound of this example was obtained in a similar yield .
mp: 43.4-43.9 °C; JR (KBr) ν: 3306, 3054, 2915, 2846, 1673, 1587, 1540, 1462, 1304, 1264, 1225, 1130, 769 $cm^{-1}$; $^1H$ NMR (80 MHz, $CDCl_3$) δ (TMS): 8.54 (broad d, J=4Hz, 1H, pyr), 7.68 (dt, $j_d$=1.6 Hz, $J_t$=7.5 Hz), 1H, pyr), 7.2 (m, 2H, pyr), 5.7 (m, 1H, NH), 5.49 (d, J=5.5 Hz), 2H, Ar-$CH_2N$), 4.2-3.8 (m, 4H, CHOCH, O=$COCH_2$), 3.7-3.3 (m, 4H, $CH_2OCH_2$), 2.2-0.7 (m, 43H).
*Analysis* calcd. for $C_{33}H_{58}N_2O_4$ : C 72.48%; H 10.69%; N 5.12%. Found: C 72.55%; H 11.00%; N 5.03%.

**EXAMPLE 21**

**(±)-*cis,trans*-2-[N-acetyl-N-[((6-((5-pentadecyl-2-tetrahydrofuranyl)methoxy)hexyl)oxy carbonyl] aminomethyl]pyridine**

Following the procedure described in example 2, but using the product obtained in example 22 instead of the one obtained in example 1, the title compound of this example was obtained in a similar yield .
IR (KBr) ν: 3455, 2921, 2850, 1737, 1700, 1589, 1567, 1462, 1429, 1389, 1367, 1341, 1285, 1206, $cm^{-1}$; $^1H$ NMR (80 MHz, $CDCl_3$) δ (TMS): 8.51 (dq, Jq=1.6Hz, Jd=5 Hz, 1H, pyr), 7.61 (dt, $J_d$=1.6 Hz, $J_t$=7.6 Hz), 1H, pyr), 7.13 (broad t, J=7.6 Hz, 2H, pyr), 5.08 (s, 2H, pyr-$CH_2$), 4.3-3.7 (m, 4H, CHOCH, O=$COCH_2$), 3.7-3.3 (m, 4H, $CH_2OCH_2$), 2.62 (s, 3H, $OCH_3$), 2.2-0.7 (m, 43H).

**EXAMPLE 22**

**(±)-*cis,trans*-2-[N-acetyl-N-[((6-((5-pentadecyl-2-tetrahydrofuranyl)methoxy)hexyl)oxy)carbonyl]-aminomethyl]-1-ethylpyridinium iodide**

Following the procedure described in example 3, but using the product obtained in example 23 instead of the one obtained in example 2, the title compound of this example was obtained in a similar yield .
IR (film) ν: 3441 ($H_2O$), 3233, 3031, 2919, 2849, 1744, 1672, 1624, 1578, 1509, 1448, 1427, 1367, 1343,

1292, 1212, 1164, 1087, 987 $cm^{-1}$; $^1H$ NMR (80 MHz, $CDCl_3$) $\delta$ (TMS): 9.67 (d, J=6Hz, 1H, pyr), 8.55 (t, J=8 Hz, 1H, pyr), 8.08 (t, J=7.6 Hz, 1H, pyr), 7.75 (d, J=7.6 Hz, 1H, pyr), 5.42 (s, 2H, pyr-$CH_2$), 5.07 (q, J=6.8 Hz), 2H, $NCH_2CH_3$), 4.5-3.7 (m, 4H, CHOCH, O=$COCH_2$), 3.7-3.3 (m, 4H, $CH_2OCH_2$), 2.64 (s, 3H, $CH_3$), 1.73 (t, 3H, $NCH_2CH_3$), 2.2-0.7 (m, 43H).

## EXAMPLE 23

**(±)-*cis,trans*-2-[N-[((5-((hexadecyloxy)methyl)-2-tetrahydrofuranyl)methoxy)carbonyl] aminomethyl] pyridine**

Following the procedure described in example 1, but using the product obtained in preparation 15 instead of the one obtained in preparation 4, the title compound of this example was obtained in a similar yield. An analitical sample of this compound can be obtained by purification by flash chromatography (ethyl acetate:hexane 4:1)

mp: 43-47° C; JR (KBr) $\nu$: 3307, 3053, 2914, 2846, 1672, 1586, 1537, 1466, 1377, 1304, 1259, 1224, 1117, 1095, 1056 $cm^{-1}$; $^1H$ NMR (80 MHz, $CDCl_3$) $\delta$ (TMS): 8.53 (broad d, J=4.5 Hz, 1H, pyr), 7.66 (broad t, J=7.6 Hz, 1H, pyr), 7.4-7.1 (m, 2H, pyr), 5.81 (m, 1H, NH), 4.54 (d, J=5.4 Hz, 2H, pyr-$CH_2$), 4.4-4.0 (m, 4H, $CHOCHCH_2OC$=O), 3.6-3.3 (m, 4H, $CH_2OCH_2$), 2.2-0.7 (m, 35H).

*Analysis* calcd. for $C_{29}H_{50}N_2O_4$ : C 70.98%; H 10.27%; N 5.70%. Found: C 70.54%; H 10.56%; N 5.75%.

## EXAMPLE 24

**(±)-*cis,trans*-2-[(N-acetyl-N-[((5-((hexadecyloxy)methyl)-2-tetrahydrofuranyl)methoxy) carbonyl] aminomethyl] pyridine**

Following the procedure described in example 2, but using the product obtained in example 23 instead of the one obtained in example 1, the title compound of this example was obtained as a colorless oil in a similar yield.

IR (film) $\nu$: 2920, 2849, 1737, 1701, 1589, 1567, 1462, 1433, 1385, 1367, 1343, 1286, 1206, 1080, 979 $cm^{-1}$; $^1H$ NMR (80 MHz, $CDCl_3$) $\delta$ (TMS): 8.49 (dq, [$J_q$=1.8Hz, $J_d$=4.5 Hz, 1H, pyr), 7.61 (dt, $J_d$=1.8 Hz $J_t$=7.6 Hz, 1H, pyr), 7.2-6.9 (m, 2H, pyr), 5.09 (s, 2H, pyr-$CH_2$), 4.3-3.9 (m, 4H, $CHOCHCH_2OC$=O), 3.5-3.2(m, 4H, $CH_2OCH_2$), 2.61 (s, 3H, $CH_3$), 2.2-0.7 (m, 35H).

## EXAMPLE 25

**(±)-*cis,trans*-2-[N-acetyl-N-[((5-((hexadecyloxy)methyl)-2-tetrahydrofuranyl)methoxy) carbonyl] aminomethyl]-1-ethylpyridinium iodide**

Following the procedure described in example 3, but using the product obtained in example 24 instead of the one obtained in example 2, the title compound of this example was obtained in a similar yield.

mp: 32-33° C; IR (KBr) $\nu$: 3457 ($H_2O$), 2919, 2849, 1747, 1684, 1624, 1579, 1509, 1447, 1376, 1212, 1162, 1087 $cm^{-1}$; $^1H$ NMR (80 MHz, $CDCl_3$) $\delta$ (TMS): 9.60 (d, J=6Hz, 1H, pyr), 8.58 (t, J=7.8 Hz, 1H, pyr), 8.09 (t, J=6 Hz, 1H, pyr), 7.90 (d, J=8 Hz, 1H, pyr), 5.45 (s, 2H, pyr-$CH_2$), 5.05 (q, J=7.4 Hz), 2H, $NCH_2CH_3$), 4.4-3.9 (m, 4H, $CHOCHCH_2OC$=O), 3.6-3.3 (m, 4H, $CH_2OCH_2$), 2.66 (s, 3H, $CH_3$), 1.73 (t, J=7.4 Hz, 3H, $NCH_2CH_3$), 2.2-0.7 (m, 35H).

*Analysis* calcd. for $C_{33}H_{57}IN_2O_5$ 1/4 $H_2O$: C 57.17%; H 8.36%; N 4.04%. Found: C 57.11%; H 8.47%; N 4.08%.

### EXAMPLE 26

**(±)-*cis,trans*-2-[N-[((5-((octadecyloxy)methyl)-2-tetrahydrofuranyl)methoxy)carbonyl] aminomethyl]-pyridine**

Following the procedure described in example 1, but using the product obtained in preparation 16 instead of the one obtained in preparation 4, the title compound of this example was obtained in a similar yield.
m.p: 53-58° C; IR (KBr) ν: 3308, 3055, 2914, 2846, 1671, 1588, 1538, 1466, 1378, 1304, 1259, 1225, 1119, 1057, $cm^{-1}$; $^1$H NMR (80 MHz, $CDCl_3$) δ (TMS): 8.54 (broad d, J = 4.5 Hz, 1H, pyr), 7.68 (broad t, J = 7.8 Hz, 1H, pyr), 7.4-7.1 (m, 2H, pyr), 5.8 (m, 1H, NH), 4.55 (d, J = 5.3 Hz, 2H, pyr-$CH_2$), 4.4-4.0 (m, 4H, $CHOCHCH_2OC=O$), 3.6-3.3 (m, 4H, $CH_2OCH_2$), 2.2-0.7 (m, 39H).

### EXAMPLE 27

**(±)-*cis,trans*-2-[N-acetyl-N-[((5-((octadecyloxy)methyl)-2-tetrahydrofuranyl)methoxy) carbonyl] aminomethyl]pyridine**

Following the procedure described in example 2, but using the product obtained in example 26 instead of the one obtained in example 2, the title compound of this example was obtained as a colorless oil in a similar yield.
IR (film) ν: 2919, 2849, 1738, 1700, 1588, 1567, 1462, 1432, 1385, 1366, 1343, 1288, 1206, 1081, 978 $cm^{-1}$; $^1$H NMR (80 MHz, $CDCl_3$) δ (TMS): 8.48 (dq, $J_q = 2$ Hz, $J_d = 5$ Hz, 1H, pyr), 7.61 (dt, $J_d = 2$ Hz, $J_t = 7.5$ Hz, 1H, pyr), 7.2-6.8 (m, 2H, pyr), 5.08 (s, 2H, pyr-$CH_2$), 4.3-3.9 (m, 4H, $CHOCHCH_2OC=O$), 3.5-3.2(m, 4H, $CH_2OCH_2$), 2.6 (s, 3H, $CH_3$), 2.2-0.7 (m, 39H).

### EXAMPLE 28

**(±)-*cis,trans*-2-[N-acetyl-N-[((5-((octadecyloxy)methyl)-2-tetrahydrofuranyl)methoxy) carbonyl] aminomethyl]-1-ethylpyridinium iodide**

Following the procedure described in example 3, but using the product obtained in example 27 instead of the one obtained in example 2, the title compound of this example was obtained as a yellow solid in a similar yield.
mp: 38-41° C; IR (KBr) ν: 3459 ($H_2O$), 2919, 2849, 1748, 1685, 1624, 1579, 1508, 1448, 1368, 1212, 1162, 1087 $cm^{-1}$; $^1$H NMR (80 MHz, $CDCl_3$) δ (TMS): 9.62 (d, J = 6Hz, 1H, pyr), 8.55 (t, J = 8 Hz, 1H, pyr), 8.10 (t, J = 6 Hz, 1H, pyr), 7.9 (d, J = 8 Hz, 1H, pyr), 5.48 (s, 2H, pyr-$CH_2$), 5.08 (q, J = 7.4 Hz), 2H, $NCH_2CH_3$), 4.4-3.9 (m, 4H, $CHOCHCH_2OC=O$), 3.6-3.3 (m, 4H, $CH_2OCH_2$), 2.66 (S, 3H, $CH_3$), 1.72 (T, J = 7.4 HZ, 3H, $NCH_2CH_3$), 2.2-0.7 (m, 39H).
*Analysis* calcd. for $C_{35}H_{61}IN_2O_5$ . 1/3 $H_2O$: C 58.97%, H 8.84%; N 3.92%. Found: C 58.66%; H 8.88%; N 3.98%.

## Claims

1) 2,5-Disubstituted tetrahydrofuran derivatives of general formula **I**.

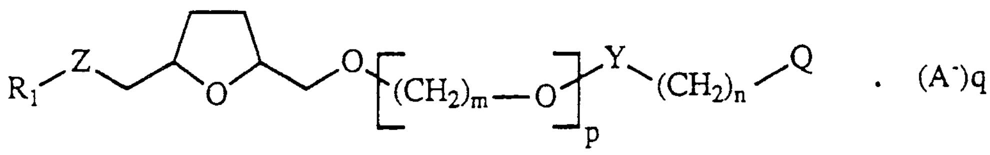

I

where:
-$R_1$ represents a $C_{10}$-$C_{24}$ alkyl, alkenyl or alkynyl group;
-Z- represents either a covalent single bond or a group of formula -O-, -C(=O)O-, -OC(=O)O-, or -$NR_2$C(=O)O-, where $R_2$ is hydrogen, $C_1$-$C_4$ alkyl, $C_1$-$C_4$ acyl, haloacetyl, or $C_1$-$C_4$ alkoxycarbonyl;
-Y- is either a covalent single bond or a -C(=O) or -C(=O)$NR_2$-group;
-n is an integer from 0 to 10;
-m is 0 or represents an integer from 2 to 8;
-p is 0 when m is 0 and p is 1 when m is not 0.
-Q means:
either a non-charged heterocyclic radical that contains a non-quaternary nitrogen atom which is linked to the alkylene chain by the ring carbon (in which case. -Q is represented by -Q′ and q = 0),
or the same radical Q′, already quaternized (in which case -Q is represented by $Q^{(+)}$ and q = 1),
or an heterocyclic radical linked to the chain by a quaternary nitrogen (in which case -Q is represented by -$Q''^{(+)}$ and q = 1).
-Q can contain additional N atoms, one or more sulphur or oxygen atoms, and can be substituted by one or several $C_1$-$C_6$ alkyl, $C_1$-$C_6$ alkoxy, carbamoyl, or $C_1$-$C_6$ hydroxyalkyl groups, or halogen.
$A^-$ represents a pharmaceutically acceptable anion, such as halide (chloride, bromide or iodide), $C_1$-$C_{10}$ alkylsulfonate, arylsulfonate or carboxylate.

2) A compound according to claim 1 wherein -$R_1$ is a linear alkyl chain of 13 to 17 carbon atoms and Z is a single bond.

3) A compound according to claim 1 wherein -$R_1$ is a linear alkyl chain of 14 to 28 carbon atoms and Z is an oxygen atom.

4) A compound according to claim 1 wherein -$R_1$ is a linear alkyl chain of 14 to 28 carbon atoms and Z is a -NHC(=O)O- group.

5) A compound according to claim 1 wherein Y is a single bond.

6) A compound according to claim 1 wherein Y is a -C(=O)- group.

7) A compound according to claim 1 wherein 11, is a -C(=O)$NR_2$-group and $R_2$ has the above mentioned meaning.

8) A compound according to claim 1 wherein m = 0 and p = 0.

9) A compound according to claim 1 wherein m is an integer from 2 to 9 and p = 1.

10) A compound according to claims 2, 5 and 8 wherein -Q, now represented by $Q'''^{(+)}$, is an heterocyclic radical linked to the alkylene chain by a quaternary Ntrogen.

11) A compound according to claims 2, 6 and 8 wherein -Q, now represented by $Q'''^{(+)}$, is an heterocyclic radical linked to the alkylene chain by a quaternary nitrogen.

12) A compound according to claims 2, 7 and 8 wherein -Q, now represented by $Q'''^{(+)}$, is an heterocyclic radical linked to the alkylene chain by a quaternary nitrogen.

13) A compound according to claims 2, 7 and 8 wherein -Q is, either an heterocycle radical that contains a non-quaternized nitrogen atom which is linked to the chain by a ring carbon, in which case -Q is represented by -Q′ and q = 0, or the same radical -Q′, already quaternized, in which case -Q is represented by $Q^{(+)}$ and q = 1.

14) A compound according to claims 3, 7 and 8 wherein -Q is, either an heterocycle radical that contains a non-quaternized nitrogen atom which is linked to the chain by a ring carbon, in which case -Q is represented by -Q′ and q = 0, or the same radical -Q′, already quaternized, in which case -Q is represented by $Q^{(+)}$ and q = 1.

15) A compound according to claims 4, 7 and 8 wherein -Q is, either an heterocycle radical that contains a non-quaternized nitrogen atom which is linked to the chain by a ring carbon, in which case -Q is represented by -Q′ and q = 0, or the same radical -Q′, already quaternized, in which case -Q is represented by $Q^{(+)}$ and q = 1.

16) A compound according to claims 2, 7 and 9 wherein -Q is, either an heterocycle radical that

contains a non-quaternized nitrogen atom which is linked to the chain by a ring carbon, in which case -Q is represented by -Q′ and q = 0, or the same radical -Q′, already quaternized, in which case -Q is represented by $Q^{(+)}$ and q = 1.

17) A compound according to claim 10 wherein $Q'''^{(+)}$ is 3-thiazolium.

18) A compound according to claim 11 wherein $Q'''^{(+)}$ is 3-thiazolium.

19) A compound according to claim 12 wherein $Q'''^{(+)}$ is 3-thiazolium.

20) A compound according to claim 13 wherein -Q′ is 2-pyridyl ($C_5H_4N$), n is 1 and $R_2$ is acetyl.

21) A compound according to claim 13 wherein $Q^{(+)}$ is 1-ethyl-2-pyridinium $(C_7H_9N)^{(+)}$, n is 1 and R2 is acetyl.

22) A compound according to claim 14 wherein -Q′ is 2-pyridyl ($C_5H_4N$), n is 1 and $R_2$ acetyl.

23) A compound according to claim 14 wherein $Q^{(+)}$ is 1-ethyl-2-pyridinium $(C_7H_9N)^{(+)}$, n is 1 and $R_2$ is acetyl.

24) A compound according to claim 15 wherein -Q′ is 2-pyridyl ($C_5H_4N$),nis 1 and $R_2$ is acetyl.

25) A compound according to claim 15 wherein $Q^{(+)}$ 1-ethyl-2-pyridinium $(C_7H_9N)^{(+)}$, n is 1 and $R_2$ is acetyl.

26) A compound according to claim 16 wherein -Q′ is 2-pyridyl ($C_5H_4N$),nis 1 and $R_2$ is acetyl.

27) A compound according to claim 16 wherein $Q^{(+)}$ is 1-ethyl-2-pyridinium $(C_7H_9N)^{(+)}$, n is 1 and $R_2$ is acetyl.

28) A compound according to claim 1 wherein the anion $A^-$ is chloride, iodide or *p*-toluenesulfonate.

29) A compound according to claim 17 wherein the product is (±)-*cis,trans*-3-[6-(5-pentadecyl-2-tetrahydrofuranyl)methoxyhexyl]thiazolium 4-methylbenzenesulfonate

30) A compound according to claim 21 wherein the product is (±)-*cis,trans*-2-[*N*-acetyl-*N*-[((5-(-(heptadecyl-2-tetrahydrofuranyl)methoxy)carbonyl] aminomethyl]-1-ethylpyridinium iodide

31) A compound according to claim 23 wherein the product is (±)-*cis,trans*-2-[*N*-acetyl-*N*-[((5(-(hexadecyloxy)methyl)-2-tetrahydrofuranyl)methoxy)carbonyl]aminomethyl]-1-ethylpyridinium iodide

32) A compound according to claim 25 wherein the product is (±)-*cis,trans*-2-[*N*-acetyl-*N*-[((5-(((N-heptadecylcarbamoyl)oxy)methyl)-2-tetrahydrofuranyl)methoxy)carbonyl]aminomethyl]-1-ethylpyridinium iodide.

33) A process for preparing 2,5-disubstituted tetrahydrofuran derivatives of general formula **I**.

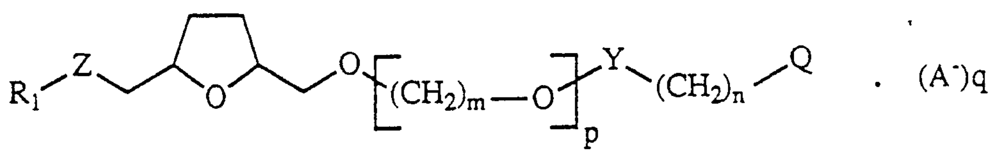

I

where:

-$R_1$ represents a $C_{10}$-$C_{24}$ alkyl, alkenyl or alkynyl group;

-Z- represents either a covalent single bond or a group of formula -O-,-C(=O)O-, -OC(=O)O-, or -$NR_2$C(=O)O-, where $R_2$ is hydrogen, $C_1$-$C_4$ alkyl, $C_1$-$C_4$ acyl, haloacetyl, or $C_1$-$C_4$ alkoxycarbonyl;

-Y- is either a covalent single bond or a -C(=O) or -C(=O)$NR_2$-group;

-n is an integer from 0 to 10;

-m is 0 or represents an integer from 2 to 8;

-p is 0 when m is 0 and p is 1 when m is not 0.

-Q means:

either a non-charged heterocyclic radical that contains a non-quaternary nitrogen atom which is linked to the alkylene chain by the ring carbon (in which case, -Q is represented by -Q′ and q = 0),

or the same radical Q′, already quaternized (in which case -Q is represented by $Q^{(+)}$ and q = 1),

or an heterocyclic radical linked to the chain by a quaternary nitrogen (in which case -Q is represented by $Q'''^{(+)}$ and q = 1).

-Q can contain additional N atoms, one or more sulphur or oxygen atoms, and can be substituted by one or several $C_1$-$C_6$ alkyl, $C_1$-$C_6$ alkoxy, carbamoyl, or $C_1$-$C_6$ hydroxyalkyl groups, or halogen.

$A^-$ represents a pharmaceutically acceptable anion, such as halide (chloride, bromide or iodide), $C_1$-$C_{10}$ alkylsulfonate, arylsulfonate or carboxylate.

caracterized in that, according to the following scheme,

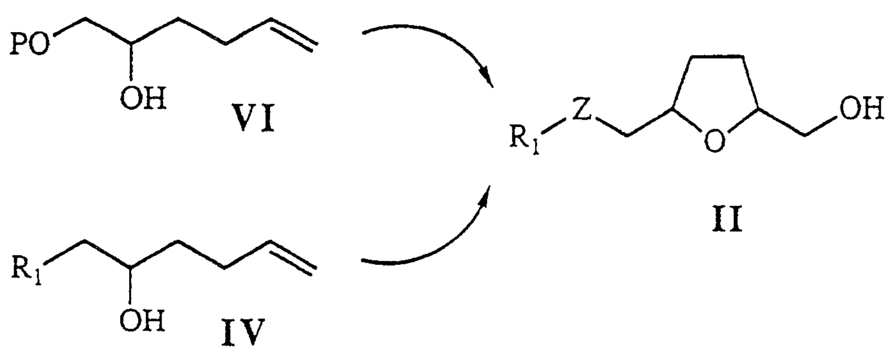

VI II IV

a) The alcohols **IV** and **VI** wherein P is a hydroxyl protecting group, are converted into a tetrahydrofuranic alcohol intermediate **(II)**, by means of epoxidation and cyclization, followed by conventional alkylation, acylation or addition-to-isocyanates reactions of the hydroxyl group (with protection deprotection when necessary).

b) When Y is -C(=O)$NR_2$-, Q is Q′ and q=0, being -Q′ an heterocyclic radical that contains a non-quaternized nitrogen atom linked to the alkylene chain by a ring carbon, the monoalcohol **II** is converted into the corresponding 2,5-tetrahydrofuran derivative, of general formula **I**, by means of the reaction with a phosgene-equivalent reagent, followed by a reaction with an amine $H_2N$-($CH_2$)-Q′ and, if desired, followed also by a conventional transformation of the carbamate funtion (such as acylation, alkylation or alkoxycarbonylation); finally, if desired, the non-quaternized nitrogen of Q′ can be quaternized by reaction with an alkylating agent to afford the corresponding quaternary ammonium salt **I** in which Y is -C(=O)$NR_2$-, Q is $Q^{(+)}$, and q=1, where $Q^{(+)}$ represents the heterocyclic radical -Q′ already quaternized, and $A^-$ is the leaving anion of the alkylating agent or another one, obtained after ionic exchange.

c) when -Q is $Q'''^{(+)}$ and q=1, where -$Q'''^{(+)}$ represents an heterocyclic radical linked to the alkyalkylene chain by a quaternary nitrogen, first of all, the monoalcohol **II** is converted into the intermediate of formula **I** with Q=B and q=0, -B being a good leaving group (or one that could be easily converted into a good leaving group by deprotection and activation of the hydroxyl group) by means of conventional reactions of ether, ester or carbamate formation (carbamates that, as above, if desired, can be alkylated, acylated or alkoxycarbonylated); and, on the other hand, the metioned intermadiate is quaternized with the neutral nitrogenated heterocycle Q‴, to afford the corresponding 2,5-disubstituted derivative of general formula **I**, in which the anion $A^-$ can be the leaving anion B-itself, or can be changed to another one by ionic exchange.

34) A process according to claim 33 where the group $R_1$ of compound **IV** represents a linear alkyl chain, and the oxidation agent is *m*-chloroperbenzoic acid, and the resulting product **II** ($R_1$ =alkyl, Z=bond) is then subsequently treated with phenylchloroformate in the presence of pyridine, then with 2-picolylamine in a chlorocarbonated solvent, then with acetyl chloride or acetic anhydride in the presence of an amine, then with ethyl iodide in acetonitrile, and finally, if desired, passed through a chloride ion exchange column to afford a compound of formula **I** where $R_1$ is a linear alkyl chain, Z is a single covalent bond, Y is -C(=O)-NC(=O)$CH_3$, n=1, p=m=0, Q= 1-ethyl-2-pyridinium, $A^-$ is chloride, and q=1.

35) A process according to claim 33 where the group P of compound **VI** is benzyl, the oxidation agent is *m*-chloroperbenzoic acid, and the resulting product **II** ($R_1$Z = PO) is treated with a linear alkyl halide or a linear alkyl isocyanate, in the presence of a base, such as sodium hydride or pyridine, respectively, and the resulting product is hydrogenated in the presence of a metal catalyst, to afford compound **II** ($R_1$ = alkyl, Z = -O- or -NHC(=O)O-), which is then subsequently treated with phenylchloroformate in the presence of pyridine, then with 2-picolylamine in a chlorocarbonated solvent, then with acetyl chloride or acetic anhydride in the presence of an amine, then with ethyl iodide in acetonitrile, and finally, if desired, passed through a chloride ion exchange column to afford a compound of formula **I** where $R_1$ is a linear alkyl chain, Z is -O- or -NHC(=O)O-, Y is -C(=O)NC(=O)$CH_3$, n=1, p=m=0, Q=1-ethyl-2-pyridinium, $A^-$ is chloride, and q=1.

36) A process according to claim 33 where the group $R_1$ of compound **IV** represents a linear alkyl chain, and the oxidation agent is *m*-chloroperbenzoic acid, and the resulting product **II** ($R_1$ =alkyl, Z=bond) is then treated with an ω-tosyloxy acid chloride or a α-tosyloxy-ω-(2-tetrahydropyranyloxy)alkane in the presence of an amine, such as pyridine or sodium hydride, respectively, and the resulting product, if necessary, is treated with an aqueous mineral acid, such as hydrochloric acid, then with *p*-toluenesulfonyl chloride in the presence of an amine, and finally reacted with a nitrogen-containing heterocycle to afford a

compound of formula **I**, where $R_1$ is a linear alkyl chain, Z is a single covalent bond, Y is -C(=O)- or -O-, n = 4-10, p=m=0, Q is a heterocycle radical directely linked to the alkylene chain by a quaternary nitrogen, $A^-$ is *p*-toluenesulfonate, and q=1.

37) A process according to claim 33 where the group P of compound **VI** is benzyl, the oxidation agent is *m*-chloroperbenzoic acid, and the resulting product **II** ($R_1Z$ = PO) is treated with a linear alkyl halide or a linear alkyl isocyanate, in the presence of a base, such as sodium hydride or pyridine, respectively, and the resulting product is hydrogenated in the presence of a metal catalyst, to afford compound **II** ($R_1$ = alkyl, Z = -O- or -NHC(=O)O-), which is then treated with an ω-tosyloxy acid chloride or a α-tosyloxy-ω-(2-tetrahydropyranyloxy)alkane in the presence of an amine, such as pyridine or sodium hydride, respectively, and the resulting product, if necessary, is treated with an aqueous mineral acid, such as hydrochloric acid, then with *p*-toluenesulfonyl chloride in the presence of an amine, and finally reacted with a nitrogen-containing heterocycle to afford a compound of formula **I**, where $R_1$ is a linear alkyl chain, Z is -O- or -NHC-(=O)-, n = 4-10, p=m=0, Q is a heterocycle radical directly linked to the alkylene chain by a quaternary nitrogen, $A^-$ is *p*-toluenesulfonate, and q=1.

38) A pharmaceutical composition comprising a compound of formula **I** in combination with a pharmaceutically acceptable carrier or diluent.

(39) The use of a compound of formula **I** for the manufacture of a medicament for the treatment of PAF-mediated illnesses.

CLAIMS FOR THE FOLLOWING CONTRACTING STATE: GR

1) A process for preparing 2,5-disubstituted tetrahydrofuran derivatives of general formula **I**.

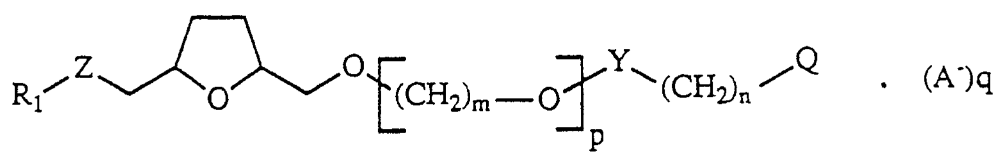

I

where:

-$R_1$ represents a $C_{12}$-$C_{24}$ alkyl, alkenyl or alkynyl group;

-Z- represents either a covalent single bond or a group of formula -O-, -C(=O)O-, -OC(=O)O, or -$NR_2$C-(=O)O-, where $R_2$ is hydrogen, $C_1$-$C_4$ alkyl, $C_1$-$C_4$ acyl, haloacetyl, or $C_1$-$C_4$ alkoxycarbonyl;

-Y- is either a covalent single bond or a -C(=C) or -C(=O)$NR_2$-group;

-n is an integer from 0 to 10;

-m is 0 or represents an integer from 2 to 8;

-pis 0 when m is 0 and p is 1 when m is not 0.

-Q means:

either a non-charged heterocyclic radical that contains a non-quaternary nitrogen atom which is linked to the alkylene chain by the ring carbon (in which case, -Q is represented by -Q′ and q=0), or the same radical Q′, already quaternized (in which case -Q is represented by $Q''^{(+)}$ and q=1), or an heterocyclic radical linked to the chain by a quaternary nitrogen (in which case -Q is represented by $Q'''^{(+)}$ and q=1).

-Q can contain additional N atoms, one or more sulphur or oxygen atoms, and can be substituted by one or several $C_1$-$C_6$ alkyl, $C_1$-$C_6$ alkoxy, carbamoyl, or $C_1$-$C_6$ hydroxyalkyl groups, or halogen.

$A^-$ represents a pharmaceutically acceptable anion, such as halide (chloride, bromide or iodide), $C_1$-$C_{10}$ alkylsulfonate, arylsulfonate or carboxylate.

caracterized in that, according to the following scheme,

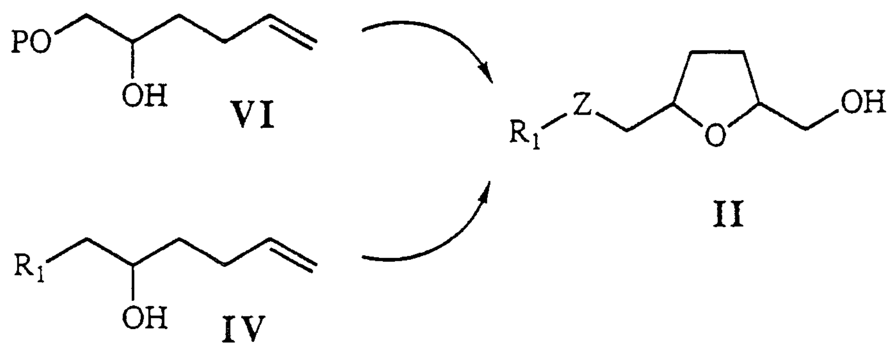

VI II IV

a) The alcohols **IV** and **VI** wherein P is a hydroxyl protecting group, are converted into a tetrahydrofuranic alcohol intermediate **(II)**, by means of epoxidation and cyclization, followed by conventional alkylation, acylation or addition-to-isocyanates reactions of the hydroxyl group (with protection.deprotection when necessary).

b) When Y is -C(=O)NR₄-, Q is Q′ and q=0, being -Q′ an heterocyclic radical that contains a non-quaternized nitrogen atom linked to the alkylene chain by a ring carbon, the monoalcohol **II** is converted into the corresponding 2,5-tetrahydrofuran derivative, of general formula **I**, by means of the reaction with a phosgene-equivalent reagent, followed by a reaction with an amine $H_2N$-$(CH_2)$-Q and, if desired, followed also by a conventional transformation of the carbamate funtion (such as acylation, alkylation or alkoxycarbonylation); finally, if desired, the non- quaternized nitrogen of Q′ can be quaternized by reaction with an alkylating agent to afford the corresponding quaternary ammonium salt **I** in which Y is -C(=O)$NR_2$-, Q is $Q^{(+)}$, and q=1, where $Q^{(+)}$ represents the heterocyclic radical -Q′ already quaternized, and $A^-$ is the leaving anion of the alkylating agent or another one, obtained after ionic exchange.

c) when -Q is $Q'''^{(+)}$ and q=1, where $Q'''^{(+)}$ represents an heterocyclic radical linked to the alkylene chain by a quaternary nitrogen, first of all, the monoalcohol **II** is converted into the intermediate of formula **I** with Q=B and q=0, -B being a good leaving group (or one that could be easily converted into a good leaving group by deprotection and activation of the hydroxyl group) by means of conventional reactions of ether, ester or carbamate formation (carbamates that, as above, if desired, can be alkylated, acylated or alkoxycarbonylated); and, on the other hand, the metioned intermadiate is quaternized with the neutral nitrogenated heterocycle Q‴, to afford the corresponding 2,5-disubstituted derivative of general formula **I**, in which the anion $A^-$ can be the leaving anion $B^-$ itself, or can be changed to another one by ionic exchange.

2) A process according to claim 1 where the group R· of compound **IV** represents a linear alkyl chain, and the oxidation agent is *m*-chloroperbenzoic acid, and the resulting product **II** ($R_1$ = alkyl, Z = bond) is then subsequently treated with phenylchloroformate in the presence of pyridine, then with 2-picolylamine in a chlorocarbonated solvent, then with acetyl chloride or acetic anhydride in the presence of an amine, then with ethyl iodide in acetonitrile, and finally, if desired, passed through a chloride ion exchange column to afford a compound of formula **I** where $R_1$ is a linear alkyl chain, Z is a single covalent bond, Y is -C(=O)-NC(=O)$CH_3$, n=1, p=m=0, Q=-1-ethyl-2-pyridirvum, $A^-$ is chloride, and q=1.

3) A process according to claim 1 where the group P of compound **VI** is benzyl, the oxidation agent is m-chloroperbenzoic acid, and the resulting product **II** ($R_1$Z = PO) is treated with a linear alkyl halide or a linear alkyl isocyanate, in the presence of a base, such as sodium hydride or pyridine, respectively, and the resulting product is hydrogenated in the presence of a metal catalyst, to afford compound **II** ($R_1$ = alkyl, Z = -O-or -NHC(=O)O-), which is then subsequently treated with phenylchloroformate in the presence of pyridine, then with 2- picolylamine in a chlorocarbonated solvent, then with acetyl chloride or acetic anhydride in the presence of an amine, then with ethyl iodide in acetonitrile, and finally, if desired, passed through a chloride ion exchange column to afford a compound of formula **I** where $R_1$ is a linear alkyl chain, Z is -O- or -NHC(=O)O-, Y is -C(=O)NC(=O)$CH_3$, n=1, p=m=0, Q=1-ethyl-2-pyridinium, $A^-$ is chloride, and q=1.

4) A process according to claim 1 where the group $R_1$ of compound **IV** represents a linear alkyl chain, and the oxidation agent is *m*-chloroperbenzoic acid, and the resulting product **II** ($R_1$ = alkyl, Z = bond) is then treated with an ω-tosyloxy acid chloride or a α-tosyloxy-ω-(2-tetrahydropyranyloxy)alkane in the presence of an amine, such as pyridine or sodium hydride, respectively, and the resulting product, if necessary, is treated with an aqueous mineral acid, such as hydrochloric acid, then with *p*-toluenesulfonyl chloride in the presence of an amine, and finally reacted with a nitrogen-containing heterocycle to afford a compound of formula **I**, where $R_1$ is a linear alkyl chain, z is a single covalent bond, Y is -C(=O)- or -O-, n

= 4-10, p = m = 0, Q is a heterocycle radical directely linked to the alkylene chain by a quaternary nitrogen, $A^-$ is *p*-toluenesulfonate, and q = 1.

5) A process according to claim 1 where the group P of compound **VI** is benzyl, the oxidation agent is *m*-chloroperbenzoic acid, and the resulting product **II** ($R_1Z$ = PO) is treated with a linear alkyl halide or a linear alkyl isocyanate, in the presence of a base, such as sodium hydride or pyridine, respectively, and the resulting product is hydrogenated in the presence of a metal catalyst, to afford compound **II** ($R_1$ = alkyl, Z = -O-or -NHC(=O)O-), which is then treated with an $\omega$-tosyloxy acid chioride or a $\alpha$-tosyloxy-$\omega$-(2-tetrahydropyranyloxy)alkane in the presence of an amine, such as pyridine or sodium hydride, respectively, and the resulting product, if necessary, is treated with an aqueous mineral acid, such as hydrochloric acid, then with p-toluenesulfonyl chloride in the presence of an amine, and finally reacted with a nitrogen-containing heterocycle to afford a compound of formula **I**, where $R_1$ is a linear alkyl chain, Z is -O- or -NHC-(=O)-, n = 4-10, p = m = 0, Q is a heterocycle radical directely linked to the alkylene chain by a quaternary nitrogen, $A^-$ is *p*-toluenesulfonate, and q = 1.

6) A process according to claim 1 wherein -R, is a linear alkyl chain of 13 to 17 carbon atoms and Z is a single bond.

7) A process according to claim 1 wherein -R, is a linear alkyl chain of 14 to 28 carbon atoms and Z is an oxygen atom.

8) A process according to claim 1 wherein -R, is a linear alkyl chain of 14 to 28 carbon atoms and Z is a -NHC(=O)O- group.

9) A process according to claim 1 wherein Y is a single bond.

10) A process according to claim 1 wherein 11, is a -C(=O)- group.

11) A process according to claim 1 wherein 11, is a -C(=O)$NR_2$- group and $R_2$ has the above mentioned meaning.

12) A process according to claim 1 wherein m = 0 and p = 0.

13) A process according to claim 1 wherein m is an integer from 2 to 8 and p = 1.

14) A process according to claims 6, 9 and 12 wherein -Q, now represented by $Q'''^{(+)}$, is an heterocyclic radical linked to the alkylene chain by a quaternary nitrogen.

15) A process according to claims 6, 10 and 12 wherein -Q, now represented by $Q'''^{(+)}$, is an heterocyclic radical linked to the alkylene chain by a quaternary nitrogen.

16) A process according to claims 6, 11 and 12 wherein -Q, now represented by $Q'''^{(+)}$, is an heterocyclic radical linked to the alkylene chain by a quaternary nitrogen.

17) A process according to claims 6, 11 and 12 wherein -Q is, either an heterocycle radical that contains a non-quaternized nitrogen atom which is linked to the chain by a ring carbon, in which case -Q is represented by -Q′ and q = 0, or the same radical -Q′, already quaternized, in which case -Q is represented by $Q^{(+)}$ and q = 1.

18) A process according to claims 7, 11 and 12 wherein -Q is, either an heterocycle radical that contains a non-quaternized nitrogen atom which is linked to the chain by a ring carbon, in which case -Q is represented by -Q′ and q = 0, or the same radical -Q′, already quaternized, in which case -Q is represented by $Q^{(+)}$ and q = 1.

19) A process according to claims 8, 11 and 12 wherein -Q is, either an heterocycle radical that contains a non-quaternized nitrogen atom which is linked to the chain by a ring carbon, in which case -Q is represented by -Q′ and q = 0, or the same radical -Q′, already quaternized, in which case -Q is represented by $Q^{(+)}$ and q = 1.

20) A process according to claims 6, 11 and 13 wherein -Q is, either an heterocycle radical that contains a non-quaternized nitrogen atom which is linked to the chain by a ring carbon, in which case -Q is represented by -Q′ and q = 0, or the same radical -Q′, already quaternized, in which case -Q is represented by -$Q^{(+)}$ and q = 1.

21) A process according to claim 14 wherein $Q'''^{(+)}$ is 3-thiazolium.

22) A process according to claim 15 wherein $Q'''^{(+)}$ is 3-thiazolium.

23) A process according to claim 16 wherein $Q'''^{(+)}$ is 3-thiazolium.

24) A process according to claim 17 wherein -Q′,, is 2-pyridyl ($C_5H_4N$), n is 1 and $R_2$ is acetyl.

25) A process according to claim 17 wherein $Q^{(+)}$ is 1-ethyl-2-pyridinium $(C_7H_9N)^{(+)}$, n is 1 and $R_2$ is acetyl.

26) A process according to claim 18 wherein -Q′,, is 2-pyridyl ($C_5H_4N$),n is 1 and $R_2$ is acetyl.

27) A process according to claim 18 wherein $Q^{(+)}$ is 1-ethyl-2-pyridinium $(C_7N_9N)^{(+)}$, n is 1 and $R_2$ is acetyl.

28) A process according to claim 19 wherein -Q′,, is 2-pyridyl ($C_5H_4N$), n is 1 and $R_2$ is acetyl.

29) A process according to claim 19 wherein $Q^{(+)}$ is 1-ethyl-2-pyridinium $(C_6H_9N)^{(+)}$, n is 1 and $R_2$ is

acetyl.

30) A process according to claim 20 wherein $-Q_n'$ is 2-pyridyl ($C_5H_4N$),nis 1 and $R_2$ is acetyl.

31) A process according to claim 20 wherein $Q^{(+)}$ is 1-ethyl-2-pyridinium $(C_6H_9N)^{(+)}$, n is 1 and $R_2$ is acetyl.

32) A process according to claim 1 wherein the anion $A^-$ is chloride iodide, or *p*-toluenesulfonate.

33) A process according to claim 4 wherein the obtained product is (±)*cis,trans*-3-[6](5-pentadecyl-2-tetrahydrofuranyl)methoxyhexyl]thiazolium 4-methylbenzenesulfonate

34) A process according to claim 2 wherein the obtained product is (±)*cis,trans*-2-[*N*-acetyl-*N*-[((5-(-(heptadecyl-2-tetrahydrofuranyl)methoxy) carbonyl]aminomethyl]lethylpyridinium iodide

35) A process according to claim 3 wherein the obtained product is (±)*cis,trans*-2-[*N*-acetyl-*N*-[((5-(-(hexadecyloxy)methyl)-2-tetrahydrofuranyl)methoxy)carbonyl]aminomethyl]-1-ethylpyridinium iodide

36) A process according to claim 3 wherein the obtained product is (±)*cis,trans*-2-[*N*-acetyl-*N*-((5-(((*N*-heptadecylcarbamoyl)oxy)methyl)-2-tetrahydrofuranyl)methoxy)carbonyl]aminomethyl]-1-ethylpyridinium iodide.

37) A process for preparing a pharmaceutical composition for treating PAF-mediated illnesses which comprises an effective amount of a compound of formula I in association with a pharmaceutically acceptable carrier, excipient or diluent therefor.